# EUROPEAN PATENT SPECIFICATION

(11) **EP 3 615 560 B1**
(45) Date of publication and mention of the grant of the patent: **05.03.2025**
(21) Application number: 18791574.9
(22) Date of filing: 26.04.2018
(51) Int. Cl.: A61K 38/18, A61P 9/10

(54) **FIBROBLAST GROWTH FACTOR FOR USE IN TREATING PATIENTS UNERGOING ISCHEMIA-REPERFUSION THERAPY**
FIBROBLASTENWACHSTUMSFAKTOR ZUR VERWENDUNG IN DER BEHANDLUNG VON PATIENTEN MIT ISCHÄMIE-REPERFUSIONSTHERAPIE
FACTEUR DE CROISSANCE DES FIBROBLASTES POUR L'UTILISATION DANS LE TRAITEMENT DE PATIENTS SUBISSANT UNE THÉRAPIE ISCHÉMIQUE DE REPERFUSION

(30) Priority: 26.04.2017 US 201762490103 P
(43) Date of publication of application: 04.03.2020
(73) Proprietor: University of Cincinnati, Cincinnati, OH 45206 (US)
(72) Inventor: SCHULTZ, Jo El J., Milford, OH 45150 (US); MOHAMMADI, Moosa, New York, NY 10016 (US)
(74) Representative: Moore, Michael Richard
(86) International application number: PCT/US2018/000110
(87) International publication number: WO 2018/200040

(56) References cited:
- WO-A2-00/13701
- WO-A2-01/98346
- US-A1- 2002 072 489
- US-B1- 6 440 934
- US-B2- 8 999 929
- GEIST ET AL.: "Combination of Enoxaparin and Fibroblast Growth Factor-1 Increases Myocardial Blood Flow and Capillary Density after Myocardial Infarction in Rabbits", EUROPEAN SURGICAL RESEARCH, vol. 37, no. 4, 1 July 2005 (2005-07-01), pages 191 - 198, XP009517491, ISSN: 0014-312X, DOI: 10.1159/000087862

## Description

### PRIORITY CLAIM

This application claims priority to U.S. Provisional application serial no. 62/490,103 filed on April 26, 2017.

### TECHNICAL FIELD

Embodiments of the invention disclosed herein relate to medical therapeutics, specifically to novel agents, and compositions useful for protecting cardiac tissue from reperfusion injury and for restoring post-infarction cardiac function.

### BACKGROUND

Myocardial infarction (MI), resulting from coronary artery disease, is a devastating event that is still one of the leading causes of morbidity and mortality worldwide. Although there have been advances in clinical therapies for coronary artery disease, more than eight million individuals suffer a myocardial infarction every year. Clinical intervention typically includes measures to immediately restore blood flow to the myocardium to prevent necrosis of the oxygen-starved tissue. Reperfusion therapy is defined as therapy to restore blood flow through a suspected or known occluded coronary artery immediately upon diagnosis and includes, for example, intravenous thrombolysis, primary angioplasty, intracoronary thrombolysis, or immediate coronary artery bypass grafting surgery.

Reperfusion results in reoxygenation of tissues that have been anoxic for some period of time. Unfortunately, it is not uncommon for reoxygenation to result in oxidative injury. Generally, the absence of oxygen and nutrients from blood during the ischemic period creates a condition in which restoration of circulation results in oxidative stress and damage. Myocardial ischemia/reperfusion (I/R) injury can result in reversible functional myocardial deterioration (i.e., stunning) and/or in irreversible tissue damage (i.e., infarction and fibrosis).

Fibroblast growth factors (FGFs) are a family of 18 ligands (FGF1-10, FGF16-23) with the mouse FGF15 being an ortholog of human FGF19. All FGF members are structurally and functionally related and are either involved in embryonic development and/or postnatal metabolism and disease. For example, a number of pharmacological and *in vitro* studies have suggested that FGFs maintain the integrity/function of the myocardium by acting directly on cardiomyocytes or indirectly via FGF angiogenic properties. However, even though a wealth of data from pre-clinical studies demonstrate that FGFs are a promising therapeutic strategy to improve myocardial survival and cardiac function, there exist several issues that complicate the clinical application of FGFs for acute myocardial infarction. One of the issues may be its interaction with heparin; exogenous heparin treatment, either with unfractionated or low molecular weight (enoxaparin) heparin, is the gold-standard medical practice for patients presenting with acute MI.

Heparin is a type of heparan sulfate (HS) made exclusively by mast cells that has the highest amount of iduronic acid and of N- and O-sulfate residues. Generally, it is acknowledged that FGFs execute pleiotropic actions by promoting FGFR dimerization and activation in a HS-dependent fashion. FGF1 binding to exogenous heparin or endogenous heparan sulfate proteoglycan (HSPG) protects it from proteases, alters its bioavailability and biodistribution, and aids in FGFR signaling. Previous reports showed that simultaneous intramyocardial injection of enoxaparin (low molecular weight heparin) combined with , such as FGF1, promoted capillary growth and regional myocardial blood flow at one week after infarction; however, Hondermarck et al. demonstrated that increasing doses of heparin co-administered with FGF1 or FGF2 weakened their binding to blood vessels in a heparin dose-dependent manner ( Experientia 1990;46:973-974). Additionally, Newman et al. (Am J Physiol Lung Cell Mol Physiol 2004;287:L191-200) showed that in rat AT2 cells, low to moderate concentrations of heparin enhanced FGF 1-mediated signals compared to FGF1 treatment alone, while a high concentration of heparin inhibited FGF1 activity (see also Fannon M, et al. Biochemistry 2000;39:1434-1445 demonstrating that in Balb/c3T3 fibroblasts, a low concentration of heparin enhanced FGF2 receptor binding, while a high concentration of heparin inhibited binding). There has been a lack of *in vivo* studies looking into the role of heparin in the cardioprotective effect of FGFs in ischemia.

Clearly, novel therapies effective to protect the myocardium from cellular damage during reperfusion therapy and to reduce acute myocardial infarction-associated mortality and morbidity remain compelling needs in the art.

### SUMMARY

Accordingly, the present inventors undertook in-depth studies to evaluate and elucidate the mechanism of the FGF - heparin interaction and applied this to the development of novel therapeutic regimens and pharmaceutical compositions, in particular for treating / substantially preventing reperfusion injury, for reducing damage caused by ischemia, and for restoring cardiac function after an ischemic event such as infarction.

The invention provides one or more Fibroblast growth factors (FGFs) for use in a method of treating or preventing cardiac reperfusion injury in a patient, the method comprising administering the one or more FGFs in conjunction with the onset of cardiac reperfusion therapy; wherein the one or more FGFs are selected from an FGF engineered to reduce heparin binding affinity, an FGF engineered to increase thermal stability, an FGF engineered to reduce both heparin binding affinity and to increase thermal stability, and combinations thereof, and wherein the FGF engineered to reduce heparin binding affinity comprises FGF^{ΔHBS}, the FGF engineered to increase thermal stability comprises FGF^{ΔTS}, and the FGF engineered both to reduce heparin binding and to increase thermal stability comprises FGF^{ΔHBS/ΔTS}.

The invention further provides one or more FGFs for use in a method for the treatment of a patient suffering from or suspected of suffering from a myocardial infarction and about to undergo or undergoing cardiac reperfusion therapy to reduce extent of the myocardial infarction, the method comprising administering the one or more FGFs to the patient in conjunction with the reperfusion therapy; wherein the one or more FGFs are selected from native FGF, FGF engineered to reduce heparin binding affinity, FGF engineered to increase thermal stability, FGF engineered both to reduce heparin binding affinity and increase thermal stability, and combinations thereof, and wherein the FGF engineered to reduce heparin binding affinity comprises FGF^{ΔHBS}, the FGF engineered to increase thermal stability comprises FGF^{ΔTS}, and the FGF engineered both to reduce heparin binding and to increase thermal stability comprises FGF^{ΔHBS/ΔTS}.

Also encompassed within the invention is a modified FGF for use in a method of treating a patient who suffered a cardiac ischemic event to restore cardiac function to a pre-ischemic event level of cardiac function, the method comprising administering the modified FGF, and, optionally, heparin, wherein the administering comprises: i)
administering one or more FGF^{ΔHBS}, or ii) administering one or more FGF^{ΔHBS} and administering one or more of FGF1, FGF2, FGF1^{ΔHBS} and FGF2^{ΔHBS}.

The references to the methods of treatment by therapy or surgery or in vivo diagnosis methods in the description or examples are to be interpreted as references to compounds, pharmaceutical compositions and medicaments of the present invention for use in those methods.

Disclosed but not claimed are methods of treating a patient undergoing ischemic cardiac reperfusion therapy, the method comprising administering a Fibroblast growth factor (FGF) in conjunction with the onset of reperfusion therapy. The FGF may be native, engineered for specific properties such as to reduce heparin binding affinity and/or to increase stability, or may comprise hybrid FGF. Recombinant FGF is preferred. FGF includes combinations of different FGF.

Also disclosed are pharmaceutical compositions comprising one or more FGFs engineered to reduce heparin binding affinity or to increase thermal stability or both, heparin, and a pharmaceutically acceptable vehicle.

Also disclosed but not claimed are methods of treating a patient undergoing cardiac reperfusion therapy following myocardial infarction to reduce extent of the myocardial infarction, the method comprising administering at least one FGF selected from native FGF, FGF engineered to reduce heparin binding affinity, FGF engineered to increase thermal stability, FGF engineered both to reduce heparin binding affinity and increase thermal stability, and combinations thereof, to the patient at the onset of reperfusion. In preferred embodiments the FGF is recombinant human (rh) FGF.

Further disclosed but not claimed are methods of treating a patient who suffered a cardiac ischemic event to restore pre-ischemic event levels of cardiac function, the method comprising administering a native or modified FGF, and, optionally, heparin. An exemplary ischemic event is myocardial infarction and exemplary cardiac functions comprise contractile and/or relaxation (systolic and diastolic, respectively) function.

These and other embodiments and aspects will be further detailed and clarified by reference to the Figures and Detailed Description, including Examples, set forth herein.

### BRIEF DESCRIPTION OF THE DRAWINGS

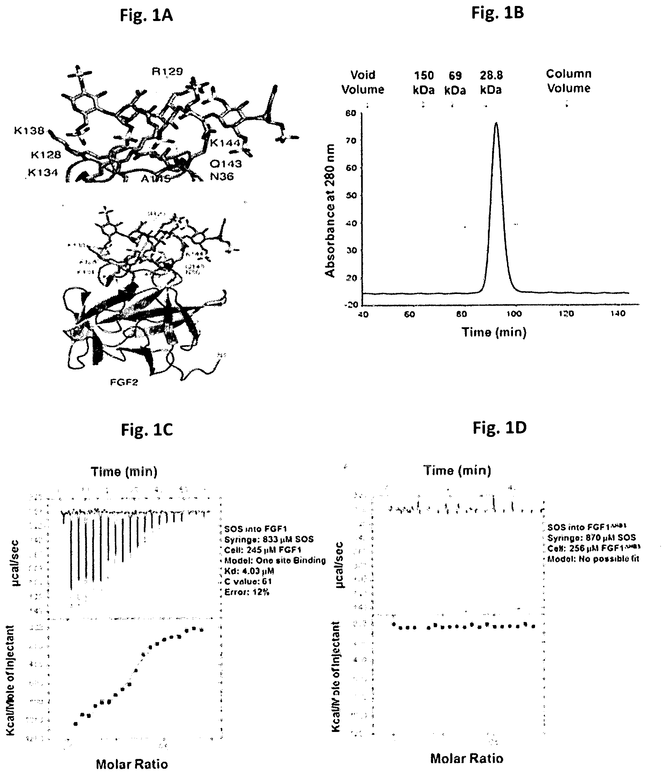

- **Figs. 1A-1D:**: Structure-guided design of the FGF 1^{ΔHBS} construct. (**Fig. 1A**) Expanded view of the hydrogen bonding interactions (dashed lines) between the HS-binding site of FGF2 and heparin hexasaccharide as observed in the crystal structure. FGF2 is shown as a ribbon diagram with HS-interacting residues rendered in sticks. The three FGF2 residues namely, K128, R129 and K134 that make important hydrogen bonding with HS are boxed. The corresponding three residues in FGF1 are K127, K128 and K133 which were mutated to aspartic acid, glutamine and valine, respectively, to engineer the FGF1_{ΔHBS} construct; (**Fig. 1B**) FGF 1^{ΔHBS} elutes as a single symmetric peak at its predicted molecular weight from a Superdex 200 sizing column. Retention times of protein standards are given above the chromatogram. (**Fig. 1C** and **Fig. 1D**) Analysis of the interactions of wildtype FGF1 and FGF1^{ΔHBS} with SOS. Indicated solutions of SOS were injected into solutions of wildtype FGF1 or FGF1_{ΔHBS} in the cell. (**Fig. 1C**) Wildtype FGF1 binds SOS with a K_{d} of 4 µM; (**Fig. 1D**) whereas, FGF1^{ΔHBS} fails to bind SOS.

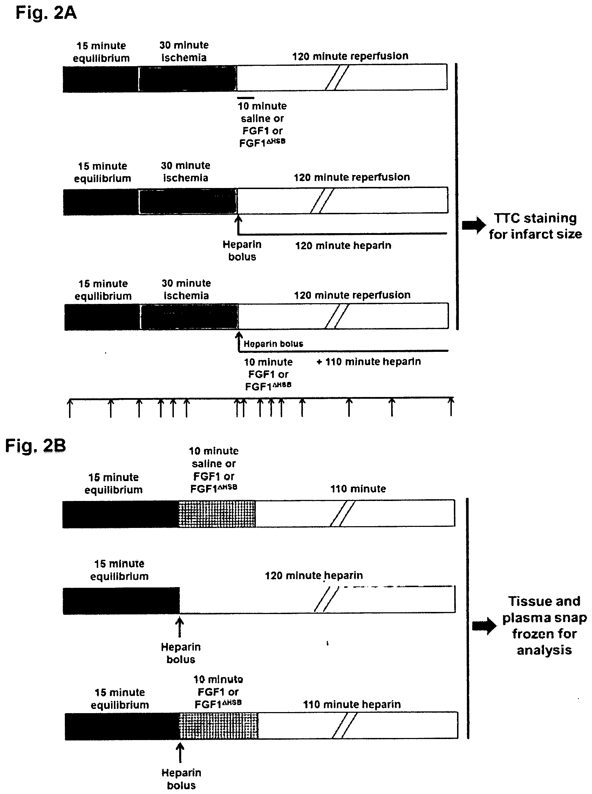

- **Figs. 2A-2B.**: Sets forth experimental design and protocols. (**Fig. 2A**) schematic of the *in vivo* ischemia-reperfusion protocol. Hemodynamic and cardiac function parameters as well as blood gases and samples (for cardiac enzyme levels and regional myocardial blood flow in pigs) are taken at specific times of baseline (equilibrium), ischemia and reperfusion. Native rhFGF or the mutated form are given during the first 15 minutes of reperfusion in the absence (-) or presence (+) of heparin. Kinase inhibition occurs 10 minutes before reperfusion. Following IR protocol, hearts are stained with TTC to evaluate infarct size; (**Fig. 2B**) Schematic of FGF1 administration protocol for analysis of the tissue distribution or signaling of FGF1 or FGF1^{ΔHBS} in the absence or presence of heparin. Solid organs and blood were collected at the point which mimics the full length of time of reperfusion for ELISA assay. The left ventricle was collected immediately post-treatment or the point which mimics the full length of time of reperfusion for FGF1 signaling.

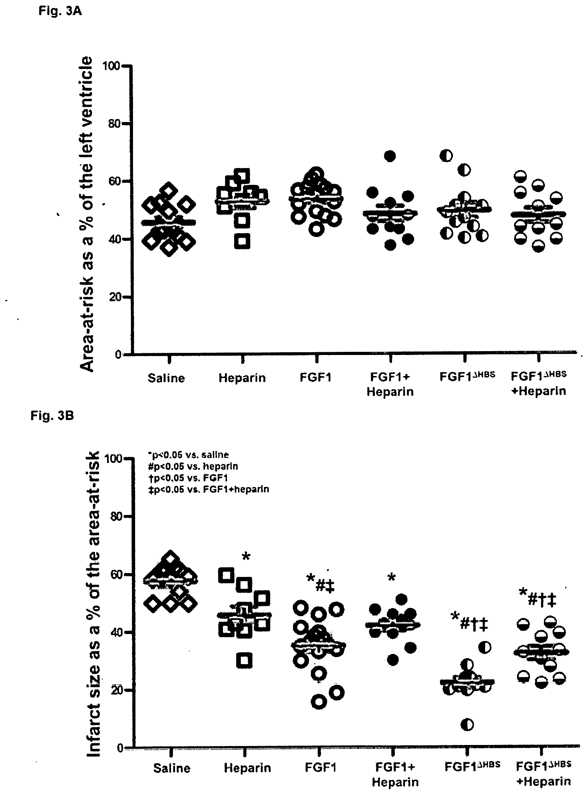

- Figs. **3A-3B.**: Shows the role of heparin in the cardioprotective effect of FGF1 on preservation of cardiomyocytes after MI. (**Fig. 3A**) Percent (%) of area-at-risk normalized to the area of the left ventricle. (**Fig. 3B**) Bar graph showing treatment with FGF 1^{ΔHBS} at reperfusion is cardioprotective. Heparin alone (second bar) 12 U/kg/hr, i.v. or FGF1 alone 10 µg/kg i.v. (third bar) at reperfusion resulted in a significant (31%) reduction in infarct size compared to saline control (first bar). Co-treatment of heparin + FGF1 ( fourth bar) lowered infarct size similar to heparin. Interestingly, the loss of heparin binding sites of FGF1 (FGF^{ΔHBS}, fifth bar) resulted in marked reduction in infarct size compared to the native form of FGF1, and this cardioprotective effect was not altered in the presence of heparin (last bar).

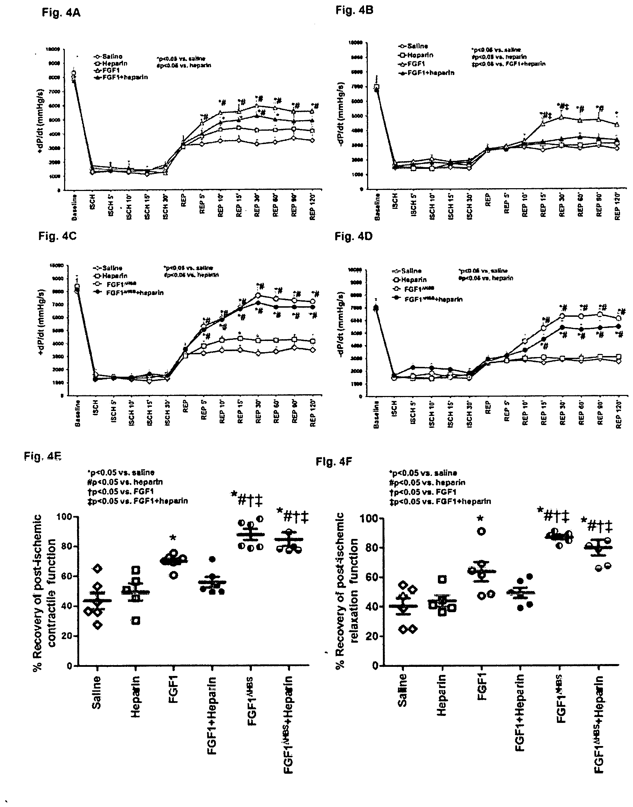

- **Figs. 4A-4F.**: Demonstrates post-ischemic recovery of contractile and relaxation function as measured by +dP/dt and -dP/dt, respectively. (**Figs. 4A** and **4C**) contractile and (**Figs. 4B** and **4D**) relaxation function of FGF1 and modified FGF1 with reduced heparin binding (FGF1^{ΔHBS}) in the absence or presence of heparin. (**Figs. 4A** and **4B**) Heparin reduced the post-ischemic improvement of cardiac function induced by FGF1; (**Figs. 4C** and **4D**) FGF1^{ΔHBS} improved post-ischemic cardiac function, even in the presence of heparin; (**Fig. 4E**) shows percent (%) recovery of contractility or (**Fig. 4F**) relaxation is +dP/dt or -dP/dt, respectively, at 120 minutes post-reperfusion normalized to baseline. *n* =5 (for heparin). *n* =6 (for saline, FGF1, FGF1+heparin, FGF1^{ΔHBS} and FGF1^{ΔHBS} + heparin). *p<0.05 vs. saline cohort, #p<0.05 vs. heparin, ‡p<0.05 vs. FGF 1+heparin. For Figs. 4A-4D the statistical test was a repeated measures 2-way ANOVA with Student-Newman-Keul post-hoc test; for Figs. 4E and 4F the statistical test performed was a 1-way ANOVA with Student-Newman-Keul post hoc test.

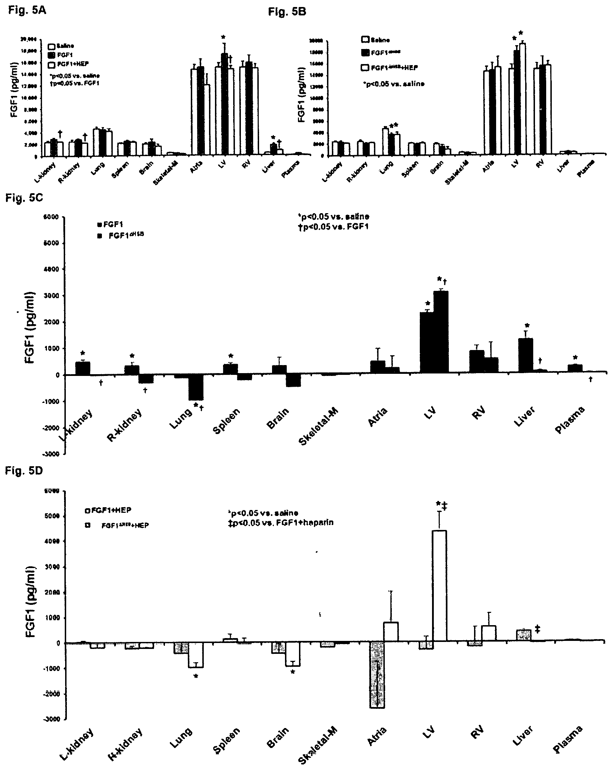

- **Figs. 5A-5D.**: Illustrates the tissue distribution of FGF1 and FGF1^{ΔHBS} in the absence or presence of heparin. (**Fig. 5A**) Total FGF1 levels (endogenous and exogenous) in saline treatment (endogenous FGF1, first bar), native FGF1 (includes endogenous + exogenous, second bar), and native FGF 1+heparin (includes endogenous + exogenous, third bar); (**Fig. 5B**) Total FGF1 levels (endogenous and exogenous) in saline treatment (endogenous FGF1, first bar), modified FGF1, FGF 1^{ΔHBS}, (includes endogenous + exogenous, second bar), and FGF1^{ΔHBS} +heparin (includes endogenous + exogenous, third bar); (**Fig. 5C**) Exogenous native FGF1 (first bar) and FGF 1^{ΔHBS} (second bar) were both significantly targeted to the heart; whereas, exogenous native FGF1 was targeted also to the kidney, spleen, liver and plasma; (**Fig. 5D**) Targeting to heart of exogenous native FGF 1+heparin (second bar) was markedly reduced compared to FGF1 alone (Fig. C) and FGF 1^{ΔHBS} +heparin (first bar). Tissue accumulation of exogenous native FGF1 or FGF 1^{ΔHBS}; exogenous FGF1 of each tissue is described as a subtraction of FGF1 concentration in native FGF 1- or FGF 1^{ΔHBS} -treated rats from that of saline treatment. L-kidney, left kidney; R-kidney, right kidney; Skeletal-M, skeletal muscle; LV, left ventricle; RV, right ventricle. n= 5 (for saline, heparin), *n=* 7 (for FGF1, FGF 1+heparin, FGF 1^{ΔHBS} +heparin, FGF1^{ΔHBS}). *p<0.05 vs. saline. †p<0.05 vs. FGF1, ‡p<0.05 vs. FGF1+heparin. The statistical test performed was 2-way ANOVA with Student Newman Keul post-hoc test.

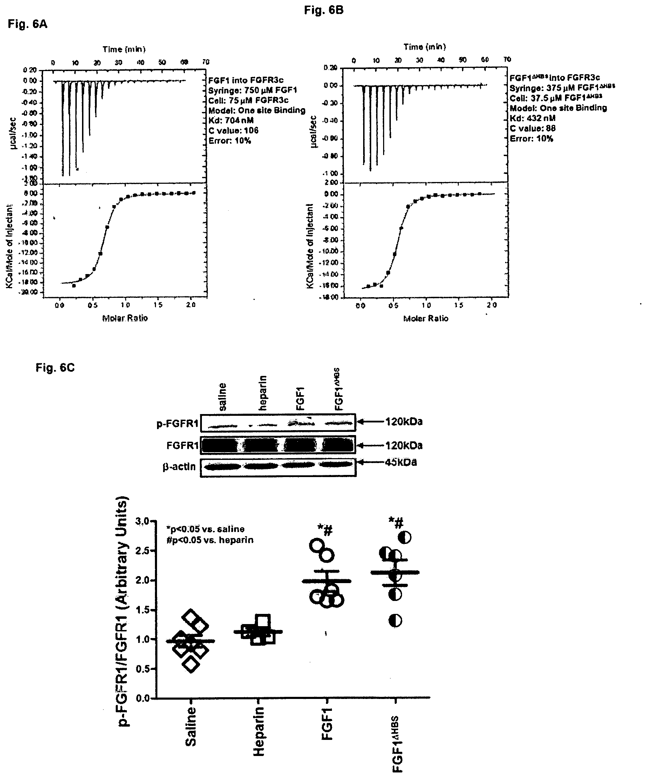

- **Figs. 6A-6C**.: Provides *in vitro* and *in vivo* characterization of the FGF 1^{ΔHBS} construct; (**Fig. 6A**) analysis of the interaction of native FGF1 and (**Fig. 6B**) FGF1^{ΔHBS} with FGFR3c; indicated solutions of wildtype FGF1 or FGF1^{ΔHBS} were injected into solutions of FGR3c ectodomain in the cell; native FGF1 and FGF1^{ΔHBS} bind FGFR3c ectodomaini with comparable affinities (704 nM verus 432 nM); (**Fig. 6C**) *in vivo* activation (i.e. phosphorylation) of FGF receptor-1in the left ventricle is similar for native FGF1 and FGF1^{ΔHBS}

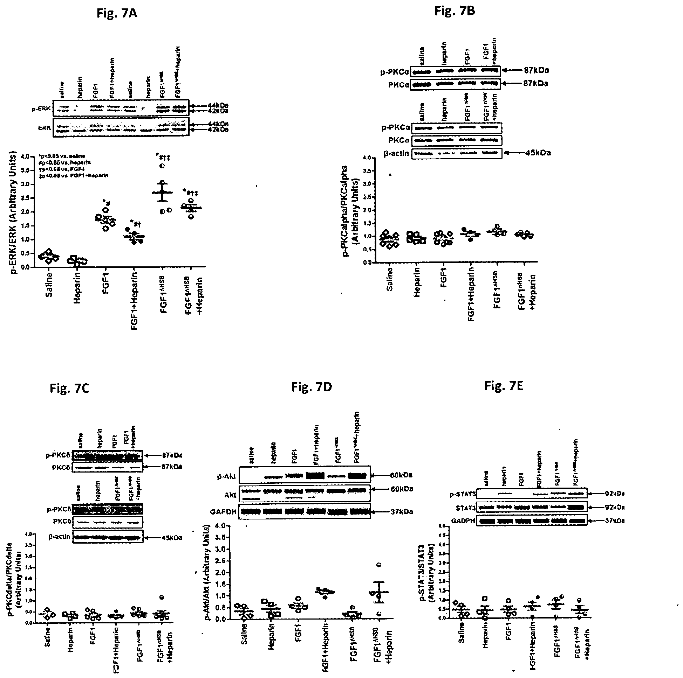

- **Figs. 7A-7E.**: FGFR signaling involved in cardioprotection. The activation (i.e., phosphorylation) of cardioprotective kinases in the RISK and SAFE pathways measured in the left ventricle collected immediately post-FGF 1 or post-modified FGF1 with reduced heparin binding (FGF1^{ΔHBS}) administration in the presence or absence of heparin. (A) ERK activation. (B) PKCa activation. (C) PKCd activation. (D) Akt activation. (E) STAT3 activation. ERK activation (panel A) was markedly increased in native FGF1 and FGF 1^{ΔHBS}, with a significantly higher level of phosphorylated ERK1/2 in the hearts from the FGF1^{ΔHBS} -treated group compared to native FGF1 treatment whether in the absence or presence of heparin. PKCalpha or delta or Akt or STAT3 activation was not different among treatment groups evaluated. Akt and STAT3 activation (panel D and panel E, respectively) were determined via an automated capillary Western blot (WES sytem) by proteinSimple. For ERK activation, n= 4-6. For PKCα activation, n= 3-8. For PKCδ activation, n= 3-7. For Akt or STAT3 activation, n=4. *P<0.05. The statistical test performed for panels A-E was Kruskal-Wallis non-parametric test with Tukey's post-hoc test.

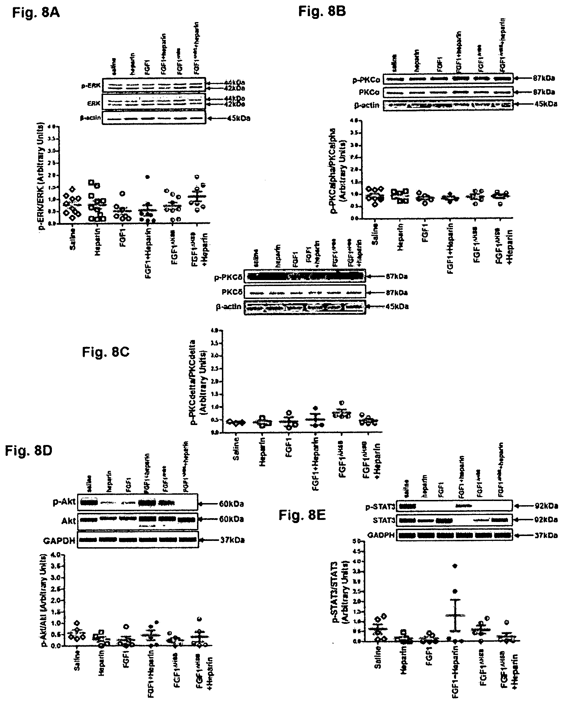

- **Figs. 8A-8E.**: FGFR signaling involved in cardio-protection. The activation (i.e. phosphorylation) of cardioprotective kinases in the RISK and SAFE pathways measured in the left ventricle collected 110-min post-FGF 1 or post-modified FGF1 with reduced heparin binding FGF1^{ΔHBS}) administration in the presence or absence of heparin. (**Fig. 8A**) ERK activation; (**Fig. 8B**) PKCa activation; (**Fig. 8C**) PKCd activation; (**Fig. 8D**) Akt activation; (**Fig. 8E**) STAT3 activation; data shows that none of ERK, PKCα, PKCδ, Akt or STAT3 activation were different among treatment groups evaluated. Akt and STAT3 activation were determined via an automated capillary Western blot (WES system) by ProteinSimple. For ERK activation, n=7-11. For PKCa activation, n= 4-8. For PKCd activation, n= 3-6. For Akt or STAT3 activation, n= 5. The statistical test performed for **Fig. 8A****-****Fig. 8E** was Kruskal-Wallis non-parametric test with Tukey's post-hoc test.

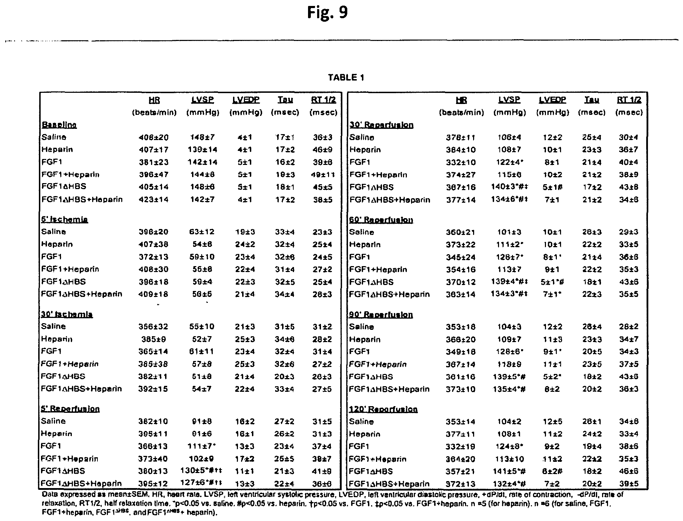

- **Fig. 9.**: **Table 1:** Cardiac parameters prior to and during I/R injury.

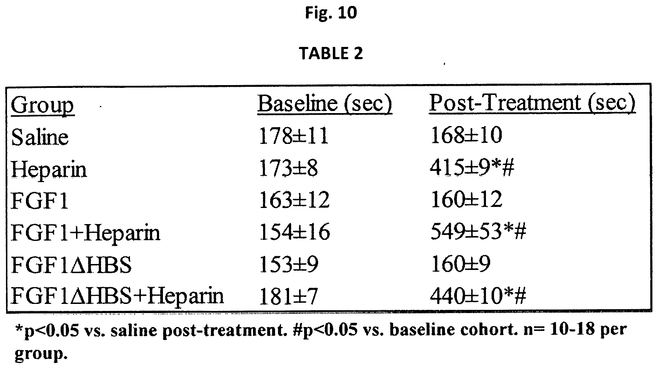

- **Fig. 10.**: **Table 2:** Blood clotting time of FGF1- and FGF 1^{ΔHBS} -treated rats in the presence or absence of heparin.

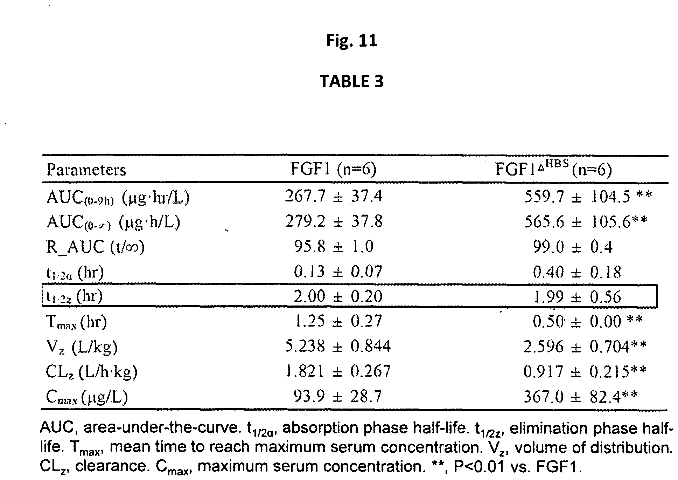

- **Fig. 11.**: **Table 3:** Pharmacokinetics of native FGF1 and FGF 1^{ΔHBS} following intraperitoneally administered treatment.

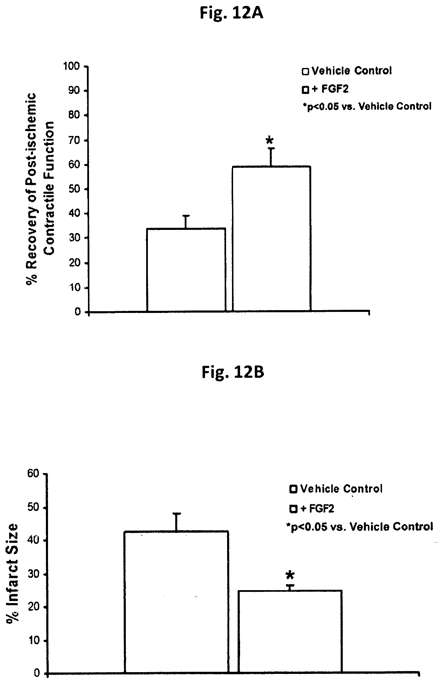

- **Figs. 12A-12B.**: Bar graphs demonstrating that FGF2 treatment at reperfusion protects the heart from post-ischemic cardiac dysfunction and myocardial infarction in an *ex vivo* model; (**Fig. 12A**) as measured by +dP/dt and (**Fig. 12B**) infarct size. Left bar is vehicle-treated and right bar is FGF2-treated wildtype mouse hearts. Admiistration of human recombinant FGF1 (10 µg) immediately at reperfusion resulted in a significant enhancement of contractile function and a significant reduction in infarct size following 60 minutes of low-flow ischemia and 60 minutes of reperfusion compared to vehicle.

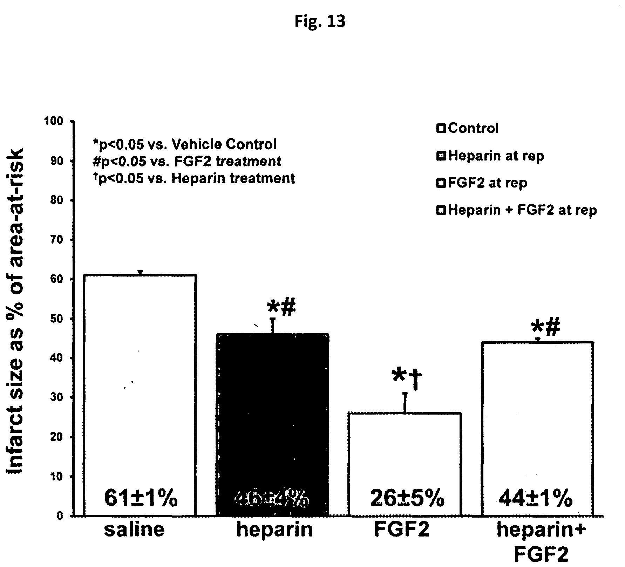

- **Fig. 13.**: Bar graph showing that FGF2 treatment at reperfusion is cardioprotecive, but was abolished with heparin co-therapy; 10 µg/kg i.v. (third bar) at reperfusion resulted in a significant (57%) reduction in infarct size compared to vehicle control (first bar); heparin treatment (second bar) at reperfusion alone lowered infarct size but not to the degree of FGF2 treatment; heparin co-therapy (fourth bar) significantly inhibited FGF2's reduction in infarct size.

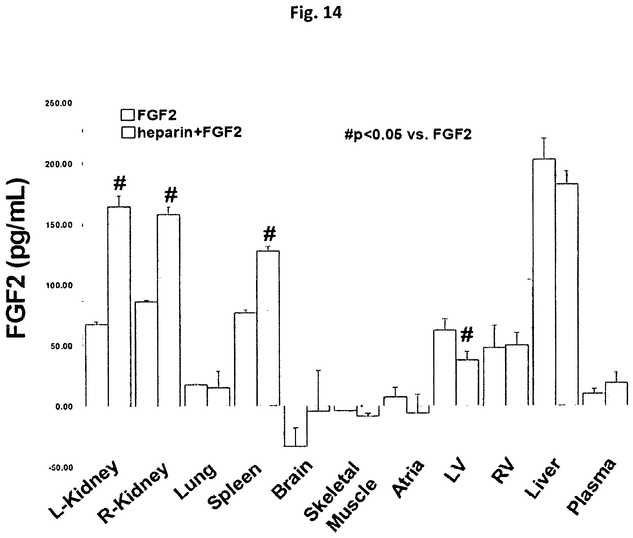

- **Fig. 14.**: Shows tissue distribution of FGF2 in the absence or presence of heparin; exogenous native FGF2 (left bar of each pair) was significantly targeted to the heart, whereas exogenous native FGF2 was targeted also to the kidney, spleen, liver, and plasma. Targeting to the heart of heparin + native FGF2 (right bar) was markedly reduced compared to FGF2 alone. Tissue accumulation of exogenous native FGF2, exogenous FGF2 of each tissue is described as a subtraction of FGF2 concentration in native FGF2-treated rats from that of saline treatment.

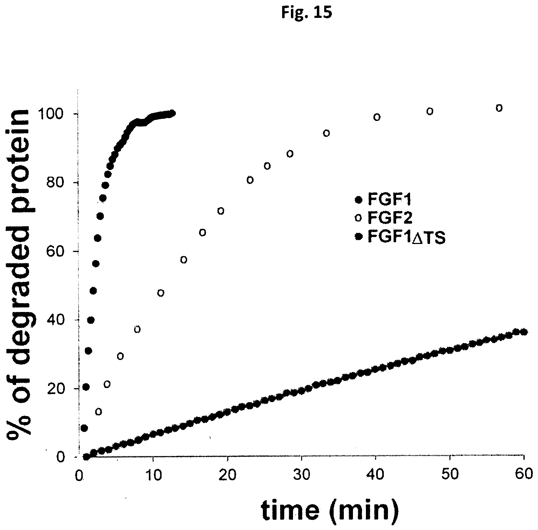

- **Fig. 15.**: Demonstrates that FGF2 and FGF 1^{ΔTS} are more resistant to proteolytic degradation than FGF1, specifically exhibiting a 7-fold increase in proteolytic resistance for FGF2 and a 20-fold increase for FGF1^{ΔTS} compared to native FGF1.

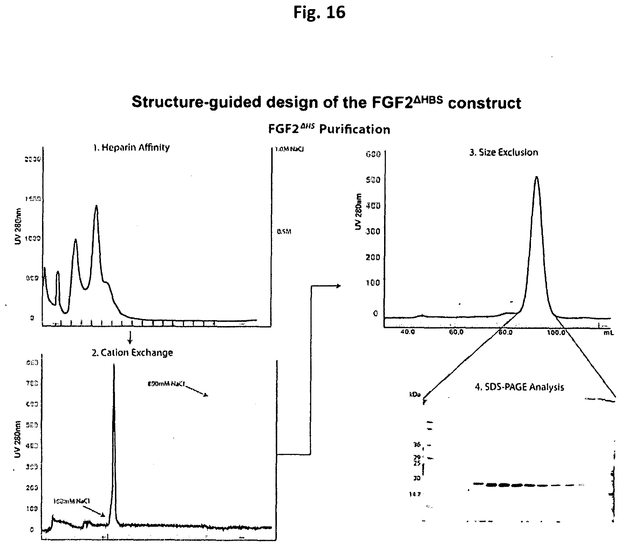

- **Fig. 16.**: Diagrammatic representation of the structure-guided design of the FGF2^{ΔHBS} construct.

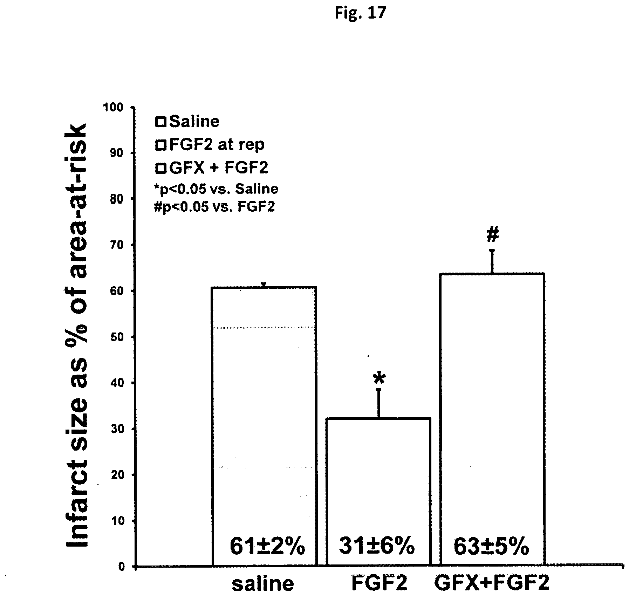

- **Fig. 17.**: Bar graph suggesting that PKC is involved in FGF2-induced cardioprotection; a non-selective PKC inhibitor, bisindolylmaleimide (GF109203X, 1 mg/kg i.v.) abolished FGF2-induced cardioprotection.

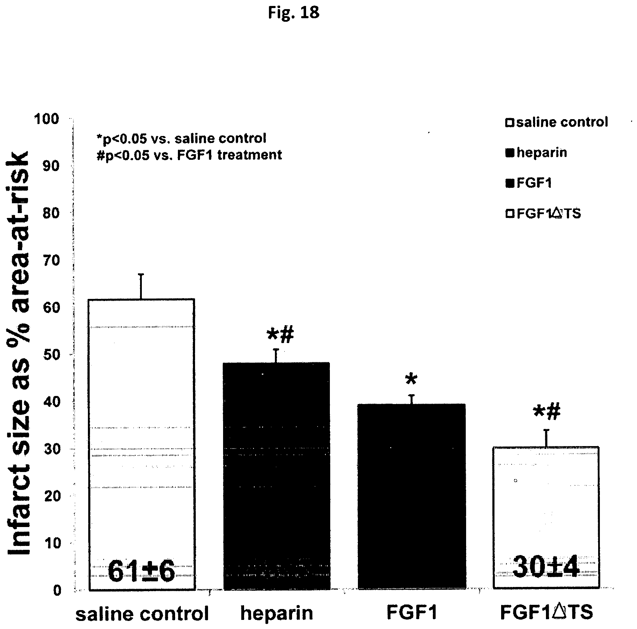

- **Fig. 18.**: Bar graphs showing that treatment with a thermally stable mutant FGF 1 (FGF1^{ΔTS}) at reperfusion is cardioprotective and results in a marked reduction in infarct size compared to native FGF1 at the same dose; heparin alone at 12 U/kg/hr, i.v. (second bar) or FGF1 alone at 10 µg/kg i.v. (third bar) at reperfusion resulted in a significant (31%) reduction in infarct size compared to saline control (first bar).

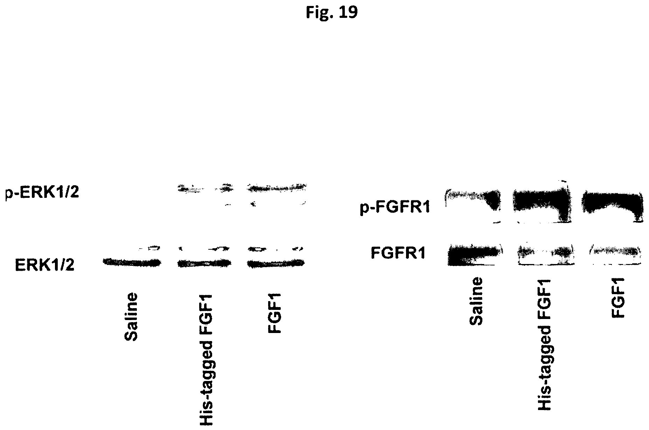

- **Fig. 19.**: Representative image of ERK activation and FGFR1 activation following 15 minutes of saline, his-tagged FGF1 and unlabled FGF1 treatment. His-tagged FGF1 and unlabeled FGF1 activated ERK (phosphor-ERK) and FGFR1 (phospho-FGRF 1) to a similar and higher level compared to salinen treatment. Total ERK and FGFR1 expression was not different between treatment groups.

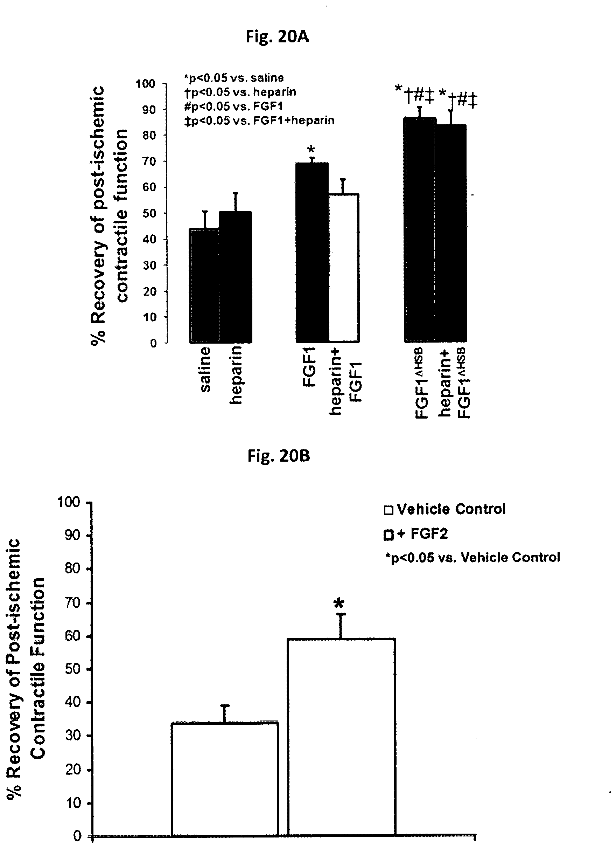

- **Fig. 20A**.: Bar graph showing that treatment at with FGF 1^{ΔHBS} at reperfusion significantly improved post-ischemic cardiac function compared to native FGF1 whether in the absence or presence of heparin. **Fig. 20B**. Bar graph showing that treatment with FGF2 at reperfusion *in vivo* protects the heart from cardiac dysfunction.

### DETAILED DESCRIPTION

Cardiovascular disease (CVD) is the primary cause of death in the United States and other industrialized nations; approximately 79 million Americans have some form of CVD with coronary (ischemic) heart disease comprising 20% of all CVD cases and 52% of all CVD deaths. Embodiments of the invention protect the myocardium from cellular damage after cardiac IR injury and promise to reduce acute MI-associated mortality and morbidity.

Heparin (unfractionated or low molecular weight form) is the standard of care for patients with MI per the guidelines of the American Heart Association and American College of Cardiology. Therefore, new cardioprotective therapeutics must demonstrate efficacy in the presence of heparin without altering heparin's anticoagulant activity. Critically, although heparin inhibits FGF1-induced cardioprotection against myocardial infarction and dysfunction,
FGF1 does not alter the desired anticoagulant activity of heparin (**Table 2,** **Fig. 3B****,** **Fig. 10**).

The biologic activity of certain Fibroblast Growth Factors (FGFs) (e.g., FGF1-FGF 10), acting at the FGF receptor (FGFR), is modulated at the cell membrane through interaction of the FGF heparin-binding region with heparin sulfate (HS) proteoglycans. Ligand bioavailability can be a significant pharmacological limitation. Further co-administration of heparins, a standard of care in the initial management of acute MI, may alter FGF pharmacokinetics and pharmacodynamics as to significantly affect their cardioprotective efficacy. FGFs have low protein stability under physiological temperatures, and therefore, which has limited their application as cardioprotective agents in the presence of heparin therapy for MI patients. For example, although FGF1 is known as a cardioprotective agent against myocardial infarction (MI), the cardioprotective efficacy of FGF1 is substantially abolished in the presence of heparin, (**Fig. 3B** and **Fig. 10**) which is a standard in the clinical treatment of ischemic events, specifically for MI patients presenting to the emergency room. The mechanism of this effect has remained largely unexplored, resulting in little interest in exploiting FGF1 for clinical applications.

Further, while several previous studies suggest that some FGFs (e.g., FGF1, FGF2) maintain integrity/function of the myocardium when administered before an ischemic insult by acting directly on cardiomyocytes or indirectly via their angiogenic properties the present investigators are the first to demonstrate the surprising finding that post-ischemic cardiac dysfunction and/or MI is significantly reduced when rhFGF2 or rhFGF1 are given after an ischemic insult in conjunction with reperfusion (just prior to, at onset, or just after onset).

Although reperfusion is necessary to reduce MI, it can also lead to further injury; therefore, a key factor to facilitate the translation of novel cardioprotective therapies into the clinical setting is the timing of administration of the cardioprotective agent. The present studies demonstrates that FGF1 or FGF1^{ΔHBS} administered just prior to or immediately upon reperfusion protected the heart from MI (**Figs. 3A-3B****,** and **Fig. 10****,** p<0.05) and LV dysfunction (**Figs. 4A-4F****,** and **Fig. 20A** p<0.05).

The FGF-heparin/heparan sulfate interactions modulate the multiple biological outcomes of FGFs, including FGF1. Heparin/heparan sulfate (HS) is implicated to prevent FGF1 from degradation and facilitate the active formation of FGF 1-FGFR complex. There is also evidence that heparin alters the bioavailability, inhibits FGF-mediated signaling as well as weakens FGF binding to tissue. The current studies demonstrate that when given immediately upon reperfusion, heparin or FGF1 alone protected the heart against myocardial infarction as evidenced by reduced infarct size and improved cardiac systolic and diastolic function at 2 hours post-ischemia (**Figs. 3A-****3B, 4A-4F, 10, and 24A** and **Table 1,** **Fig. 9**)**.**

The present inventors employed a modified FGF with reduced heparin-binding affinity (FGF1^{ΔHBS}) (**Figs. 1C** and **1D**), which is still compatible with FGFR binding and signaling (**Figs. 6A-6C**), in the treatment of MI. In the studies set forth herein, heparin had little inhibitory effect on FGF 1^{ΔHBS} compared to native FGF1 as measured by the preservation of cardiac survival and muscle function (**Figs. 3A-3B** and **Figs. 4A-4F****,** respectively, and **Figs. 24A** and **24B**).

Furthermore, similar to native FGF1, FGF 1^{ΔHBS} targeted largely to the heart (2282±97 pg/mL vs. 3073±101 pg/mL, respectively) compared to other tissue types (**Fig. 5C**); however, unlike native FGF1, which in the presence of heparin co-treatment led to a re-distribution away from the heart, FGF1^{ΔHBS} even with heparin administration still directed mostly to the heart as FGF1^{ΔHBS} alone (4336±775 pg/mL vs. 3073±101 pg/mL, respectively) compared to other organs evaluated (see **Figs. 5C** and **5D**). Although FGF1^{ΔHBS} demonstrates reduced heparin/heparin sulfate-binding, it still accumulates significantly to the heart like its native form.

The heparin-binding site of FGF is distinct from the FGF receptor (FGFR) binding site (Bombardini T, et al. Angiology 1997;48:969-976). The ITC data (**Figs. 6A** and **6B**) show that the HS-binding site mutations do not impact FGFR binding ability of FGF 1^{ΔHBS} (i.e., retains normal receptor binding affinity), and confirm that the HS mutations have no adverse effect on tertiary folding of the ligand. Moreover, the *in vivo* data (**Fig. 6C**) demonstrate that the mutant FGF1 (FGF1^{ΔHBS}) activates (as measured by phosphorylation status) downstream FGF signaling (e.g., ERK) (**Fig. 7A**), providing further evidence that normal receptor binding activity is preserved *in vivo* with FGF 1^{ΔHBS}. Although it is well-documented that binding to heparan sulfate aids with the proper presentation of FGFs to FGFRs and formation of stable FGF/FGFR complexes and signaling, this appears to not be the case for the cardioprotective activity of FGF1^{ΔHBS}. Even in the absence of heparin binding, FGF1^{ΔHBS} activated FGFR1 and ERK signaling in the heart, possibly due to the elevated concentration of FGF1^{ΔHBS} to the heart. This enhanced affinity to the receptor, and higher ERK activation of FGF1^{ΔHBS} suggests that in conjunction with the re-distribution of FGF 1^{ΔHBS} to the heart, elevated ERK signaling may be the mechanism by which FGF 1^{ΔHBS} elicits a greater protection against ischemia-reperfusion injury.

Surprisingly, there was no difference in activation of the other cardioprotective kinases studied, including PKC, Akt or STAT3 of which the latter two kinases are involved in RISK and SAFE pathways of cardioprotection and post-conditioning (**Figs. 7B-7D**). Normally, FGF1 activates FGFR which is coupled to intracellular signaling pathways including the RAS-MAPK, PI3K-Akt, PLCα-PKC, and STAT pathways. The stimulated receptor then recruits and activates several docking proteins containing src homology (SH-2) domains, e.g., Phospholipase C (PLC) γ and Shb, or phosphotyrosine binding domains, e.g., SHC and FRS2 (FGFR substrate 2). The phosphorylation of the phosphotyrosine site, Y-654, is elevated in the presence of FGF1 or FGF 1^{ΔHBS} (**Fig. 6C**), which leads a binding complex of SHC-FRS2-GRB2-SOS-RAS-Raf-1 and activation of MAPK signaling. In the disclosed study, FGF1^{ΔHBS} triggers increased ERK activation, but not PKC, Akt or STAT3 signaling.

Examples set forth herein also demonstrate design and development of additional embodiments of modified FGFs, assess their pharmacokinetic (PK)/pharmacodynamics (PD) properties *in vitro,* and further show i.v. administration of native and mutant FGF1, FGF2, FGF4, or FGF5, and confirmation that manipulation of heparin sulfate/heparin-binding or the protein stability of selected FGFs enhances delivery/targeting to the heart. While simultaneous heparin and native FGF administration at reperfusion diminished the cardioprotective effect of FGF, heparin had little effect on the modified FGFs activity. The advent of recombinant human (rh) fibroblast growth factor (FGF) as potential cardioprotective therapy is disclosed and exploited herein.

Ligand bioavailability at the target site of action is a significant limitation for an intravenous FGF therapeutic in acute MI because of FGF's heparin-binding properties. Most intravenously administered FGF is expected to be cleared by heparan sulfate (HS) proteoglycans ubiquitously expressed in all tissues/organs, thus limiting FGF bioavailability at cardiac tissue sites, which is a reflection of FGF-HS proteoglycan interactions. It was previously unknown as to whether heparin therapy for acute MI would alter FGF-induced cardioprotection.

An *in vivo* rat model of myocardial infarction was used to assess whether FGF2 is cardioprotective in a clinically relevant setting of acute MI. RhFGF2 treatment (10µg/kg, i.v.) at reperfusion resulted in a significant (70%) reduction in infarct size compared to controls (**Fig. 13**). Heparin treatment, at reperfusion alone, reduced infarct size somewhat, but not to the degree of FGF2 treatment (p<0.05); and the heparin-induced reduction of myocardial infarct was similar to that previously reported. Notably, heparin co-administration significantly inhibited rhFGF2-induced cardioprotection against MI (**Fig. 13**). The area-at-risk was not different among the groups (data not shown). Similar to **Fig. 12A****,** rats treated with FGF2 recovered to 68% of its baseline left ventricular (LV) developed pressure compared to saline treatment (51%). Other LV measures show that rat hearts treated with FGF2 also have improved relaxation compared to saline treatment (data not shown). Based on these results, modification of FGFs' interaction with heparin, for example by mutation of the FGF heparin-binding region, provides a novel approach to enhancing FGF-induced cardioprotection.

Another significant pharmacological limitation to therapeutic potency and efficacy is the protein instability of FGFs at physiological temperatures. Using the rat model of myocardial infarction, the Examples herein demonstrate that the same dose (10µg/kg) of a stable FGF1 mutant (FGF1^{ΔTS}) was more efficacious in reducing infarct size compared to the same dose of native FGF1 (see **Fig. 18**).

FGFs (e.g. FGF1, FGF2, FGF4, and FGF5) implicated in cardioprotection via acute (non-mitogenic) or chronic (angiogenic) mechanisms exhibit high affinities for HS, and therefore, when administered *in vivo,* these FGFs are immobilized by abundantly expressed HS on endothelial cells. This reduces the effective concentration of these FGFs in heart tissue. Also, pharmacological applications of FGFs are limited due to its low stability at physiological temperatures. The present investigators demonstrate that therapeutic efficacy is improved by manipulating pharmacokinetic properties, e.g., reducing their affinity for HS and/or enhancing the thermal stability. Structural data on FGF-HS interaction provided a framework which allowed for manipulation of HS binding affinity and/or protein stability of these FGF to improve their biodistribution and bioavailability without interfering in their ability to activate cardiac FGF receptors.

Based on crystallographic data, three basic residues at the HS binding site of FGF2, namely, K128, R129, and K134, mediate the majority of hydrogen bonding with HS (Fig. 1A). Modeling studies demonstrated that the corresponding three residues in FGF1, FGF4 and FGF5 would also play key roles in HS binding. According to a structural analysis, mutations of these three HS-binding residues should reduce the HS-binding affinity of these ligands to a degree that is still compatible with HS-mediated FGF-FGFR binding and dimerization. An FGF1 mutant referred to herein as FGF1^{ΔHBS} was previously designed to carry the K127D/K128Q/K133V triple mutation in its HS-binding site. FGF1^{ΔHBS} was expressed in E. coli and purified to homogeneity using anion exchange chromatography followed by size exclusion chromatography. FGF1^{ΔHBS} elutes as a monodisperse peak at the predicted retention time, indicating that the mutations do not harm the tertiary folding of the ligand (**Fig. 1B**). K127, K128, and K133 are surface-exposed and do not play any role in ligand folding. To test the impact of mutations on HS binding ability of FGF1, isothermal titration calorimetry (ITC) was used to compare interactions of native FGF1 and FGF1^{ΔHBS} with Sucrose Octasulfate (SOS, a known surrogate for HS). As shown in **Fig. 1C****,** native FGF1 bound SOS with a Kd of 4µM; whereas FGF1^{ΔHBS} binding to SOS was negligible (**Fig. 1D**), demonstrating that the FGF 1^{ΔHBS} mutant sustained a substantial loss in HS binding.

The heparin-binding site of FGF is distinct from the FGF receptor (FGFR) binding site. Nonetheless, to ensure that mutations in the heparin-binding site will not impair FGF binding to its receptor, isothermal titration calorimetry (ITC) was used to compare the binding interactions of native FGF1 and FGF 1^{ΔHBS} with comparable affinities to FGFR3c ecodomain show that the HS-binding site mutations do not impact FGFR binding ability of FGF1 (**Fig. 6A-6C**)**.** Importantly, ITC data showing that FGF 1^{ΔHBS} retains normal receptor binding affinity confirm that the HS mutations have no adverse effect on tertiary folding of the ligand. Moreover, in vivo data demonstrate that mutant FGF1 activates (as measured by phosphorylation status) the FGF receptor (**Fig. 6C**) as well as downstream FGF signaling (**Fig. 8A**), providing further evidence that normal receptor binding activity is preserved in vivo with mutant FGF1.

The same strategy as for FGF1 was utilized to develop and confirm efficacy for FGF2, FGF4, and FGF5 mutants with reduced HS-binding affinity.

Low stability is considered to be a major factor that can limit pharmacological applications of proteins. Since many FGFs, including FGF1, FGF4, and FGF5, are partially unfolded at physiological temperatures, modifications to improve protein stability and reduce protein unfolding, aggregation and/or proteolytic degradation were designed to increase *in vivo* efficacy as therapeutics in IR injury. A homology approach and rational design strategy was developed by the present inventors and several highly stable variants of FGF1 (single and multiple) exhibiting significantly increased denaturation temperature and up to ten-fold prolonged half-life were constructed. Crystallographic structures of the FGF1 thermal stable mutant, termed FGF1^{ΔTS}, revealed only three localized conformational changes within the protein structure. The significant change in FGF1 stability resulted from the formation of a short 3₁₀-helix (position 550, an improvement in the propensity of amino acids to form β-sheets (position 620 and the rearrangement of a local hydrogen-bond network (positions 62 and 108). To design highly stable variants of FGF2, FGF4, and FGF5, the consensus concept was applied (see J. Mol. Biol. 2005;352:860-875 and Protein Eng. Des. Sel 2004; 17:603-611). Residues involved in receptor binding were excluded from the pool of potential substitutions. FGF structures available in the RCSB Protein Database were examined to define the precise location of secondary structures and estimate possible improvement of secondary structure elements using β-sheet and β-turn amino acid potentials for preselected mutations.

The structural region of FGF ligands involved in conformational stability is also distinct from the FGF receptor binding site as no displacement of residues involved in receptor binding was observed. Published data shows that the thermally stable mutant of FGF1 (FGF1^{ΔTS} containing Q55P, S62I, and H108G FGF 1-stabilizing mutations) activates FGF receptors (i.e., ERK activation, cell proliferation) greater than the native FGF1, indicating that mutations to enhance protein stability do not interfere with FGF receptor signaling. To confirm that FGF^{ΔTS} mutants bind properly to FGFRs and are able to induce signal transduction, SPR measurements were performed using recombined extracellular domain of FGFRs and stabilized FGFs. In the SPR experiments, individual recombinant FGFR were covalently immobilized on a carboxymethylated dextran sensor chip using an amine coupling on the level <500 response units. FGF^{ΔTS} mutants were injected as analyte on the sensor chip at increasing concentrations to determine the affinity constants. Binding of FGF^{ΔTS} mutants to cells expressing specific FGFRs were monitored using Ligand Tracer system. Interaction of fluorescently-labeled FGF mutants with FGF receptors on the cell surface were monitored directly in culture. Proper binding of FGF^{ΔTS} mutants was verified by a competitive binding assay using radio-labeled native FGF proteins.

Some embodiments of the invention based on the aforementioned studies provide one or more fibroblast growth factors for use in methods for treating patients suffering from ischemia and/or having experienced an ischemic event and who are either undergoing or scheduled to undergo reperfusion therapy. Embodiments comprise administering one or more fibroblast growth factors (FGFs) in conjunction with the onset of reperfusion therapy. In the context of the claims, "in conjunction with" means within the same therapeutic time frame, for example at any time between 2 hours prior to onset, at onset, to 2 hours subsequent to onset of reperfusion therapy, inclusive of all time points contained therein. More than one dose is also contemplated. According to specific embodiments, the ischemia has proceeded to cell injury and is an ischemic event, and in some specific embodiments the ischemia has proceeded to necrosis and is an ischemic event, for example, a myocardial infarction.

It is understood throughout this disclosure that mutant FGF includes recombinant human (rh) FGF within the scope thereof. Medicinal grade biologics are typically manufactured as recombinant species, and studies disclosed herein confirm that artifacts of recombinant technology do not alter the pharmacodynamics or desired properties of the FGFs.

In humans, 22 FGFs are known and constitute a structurally related family of signaling proteins involved in a wide variety of cellular processes. A defining property of FGFs is that they bind to heparin and heparin sulfate and they are therefore found in association with heparan sulfate proteoglycans and released locally upon injury or remodeling, including angiogenic remodeling. Thus, any FGF is contemplated including engineered FGF, recombinant forms, and hybrids. Combinations of species of FGF are contemplated.

According to the embodiments, the FGF is selected from a recombinant human FGF engineered to reduce heparin binding affinity (FGF^{ΔHBS}), an rhFGF engineered to increase thermal stability (FGF^{ΔTS}), an rhFGF engineered to reduce both heparin binding affinity and to increase thermal stability (FGF^{ΔHBS/ΔTS}), and combinations thereof. Particular such FGF mutants are known in the art, for example, design and construction of low molecular weight and high molecular weight FGF 1^{ΔHBS} are disclosed in U.S. Patent No. 9925241, and design and construction of FGF^{ΔTS} are disclosed in Zakrzewska M. et al. (J. Mol. Biol. Vol. 352(4); 2005 Sept., 860-875) (Protein Eng. Des. Sel. 17(8) 2004 Aug, 603-11).

According to even more specific embodiments, the FGF^{ΔHBS} comprises one or more of FGF1^{ΔHBS}, FGF2^{ΔHBS}, FGF4^{ΔHBS}, and FGF5^{ΔHBS}. According to other specific embodiments, the FGF^{ΔTS} comprises one or more of FGF 1^{ΔTS}, FGF2^{ΔTS} , FGF4^{ΔTS} , and FGF5^{ΔTS}, and the FGF^{ΔHBS/ΔTS} comprises one or more of FGF1^{ΔHBS/ΔTS}, FGF2^{ΔHBS/ΔTS} FGF4^{ΔHBS/ΔTS}, and FGF5^{ΔHBS/ΔTS}. Embodiments of FGF2, FGF4 and FGF5 mutants were affirmatively designed and tested by the inventors for efficacy in the inventive methods. Treatment may or may not include co-administering Heparin. In specific embodiments, treatment further comprises co-administration with Heparin, and in very specific embodiments the Heparin is unfractionated or low-molecular weight. In the context of the invention, "co-administering" is defined as administering contemporaneously or in tandem in the same therapeutic time frame. "Contemporaneously" comprises administering as a single dosage form or in multiple dosage forms. According to specific embodiments "administering comprises systemically administering via an enteral or parenteral route. According to very specific embodiments the dosage form comprises and injectable dosage form and administering comprises intravenously administering. Administering in tandem comprises any order and frequency as necessary or desired, including without limitation administering the FGF followed by administering Heparin, or administering Heparin followed by FGF, or any combination thereof. According to very specific embodiments, administering in tandem comprises administering Heparin prior to the onset of reperfusion therapy and administering FGF at the onset of reperfusion therapy, optionally, followed by administering one or more doses of heparin during or after reperfusion therapy.

Other embodiments are directed to pharmaceutical compositions comprising one or more FGFs engineered to reduce heparin binding affinity or to increase thermal stability or both, and a pharmaceutically acceptable vehicle. In other specific embodiments, the pharmaceutical compositions further comprise heparin. In very specific embodiments, the FGF engineered to reduce heparin binding affinity comprises FGF^{ΔHBS}, the FGF engineered to increase thermal stability comprises FGF^{ΔHBS} and the FGF engineered both to reduce heparin binding and to increase thermal stability comprises FGF^{ΔHBS/ΔTS}. According to even more specific embodiments, the FGF^{ΔHBS} is selected from one or more of FGF1^{ΔHBS}, FGF2^{ΔHBS}, FGF4^{ΔHBS}, and FGF5^{ΔHBS}, the FGF^{ΔTS} is selected from one or more of FGF1^{ΔTS}, FGF2^{ΔTS}, FGF4^{ΔTS}, and FGF5^{ΔTS}, and the FGF^{ΔHBS/ΔTS} is selected from one or more of FGF 1^{ΔHBS/ΔTS}, FGF2^{ΔHBS/ΔTS} FGF4^{ΔHBS/ΔTS}, and FGF5^{ΔHBS/ΔTS}. In some specific embodiments, where included, Heparin is selected from unfractionated or low molecular weight Heparin. In all embodiments reciting native or engineered FGF, recombinant human (rh) FGF is contemplated, and rhFGF may include, for example, tags that are artefactual to recombinant technology, exemplified by his-tags.

Another embodiment is directed to one or more FGF for use in methods of treating a patient who suffered or is suspected of suffering from a myocardial infarction and about to undergo or undergoing cardiac reperfusion therapy to reduce extent of the myocardial infarction. Embodiments comprise administering at least one FGF selected from FGF engineered to reduce heparin binding affinity, FGF engineered to increase thermal stability, FGF engineered both to reduce heparin binding affinity and increase thermal stability, and combinations thereof, to the patient in conjunction with the reperfusion therapy.

In the context of the claims, "in conjunction with" means within the same therapeutic time frame, for example at any time between 2 hours prior to onset, at onset, to 2 hours subsequent to onset of reperfusion therapy, inclusive of all time points contained therein. More than one dose within a therapeutic timeframe is also contemplated. A person of ordinary skill in the art understands that specific dosing schedules may vary with patient age, gender, size, tolerance to injections, co-morbidities, and the like. Infarct extension is a progressive increase in an amount of myocardial necrosis within the infarct zone of the original MI and can manifest as an infarction that extends and involves adjacent myocardium or as a sub-endocardial infarction that becomes transmural. Clinical means for assessing extent in a living, conscious patient include assessing serial changes in enzyme activity, for example in serum creatine phosphokinase (CPK) activity , imaging such magnetic resonance imaging, and echocardiographic methods. A reduction in "extent" of myocardial infarction is based on a comparison to an expected extent based on population averages specific for a patient's age, gender and medical history and such will be apparent to a clinician of ordinary skill in the art.

According to specific embodiments, the FGF engineered to reduce heparin binding affinity comprises FGF^{ΔHBS}, the FGF engineered to increase thermal stability comprises FGF^{ΔHBS}, and the FGF engineered both to reduce heparin binding and to increase thermal stability comprises FGF^{ΔHBS/ΔTS}. According to even more specific embodiments, the FGF^{ΔHBS} is selected from one or more of FGF 1^{ΔHBS} FGF2^{ΔHBS} FGF4^{ΔHBS}, and FGF5^{ΔHBS.} the FGF^{ΔTS} is selected from one or more of FGF1^{ΔTS}, FGF2^{ΔTS} , FGF4^{ΔTS} and FGF5^{ΔTS}; and the FGF^{ΔHBS/ΔTS} is selected from one or more of FGF1^{ΔHBS/ΔTS}, FGF2^{ΔHBS/ΔTS} FGF4^{ΔHBS/ΔTS}, and FGF5^{ΔHBS/ΔTS}. According to some specific embodiments the FGF may be used in a method of treating further comprises co-administering Heparin. According to very specific embodiments, the Heparin comprises an unfractionated or low-molecular weight heparin.

Another embodiment is directed to one or more FGF for use in methods of treating a patient who suffered a cardiac ischemic event to restore a level of pre-ischemic event cardiac function, the method comprising administering one or more FGF selective from modified FGF, and, optionally, heparin as defined in the claims. It is well-known that cardiac function is typically permanently compromised in patients who suffer infarction. Restoring a level of pre-ischemic event cardiac functioning comprises, for example, restoring heart muscle contractile (systolic) or relaxation (diastolic) function to greater than 40%, greater than 50%, greater than 60%, greater than 70%, or greater than 80% of pre-infarction or other baseline cardiac function. In very specific embodiments, cardiac function is restored to between about 85% and 90% of pre-infarction cardiac function. According to some specific embodiments, the modified FGF comprises FGF^{ΔHBS}. According to other specific embodiments the modified FGF comprises one or more of FGF1, FGF2, FGF 1^{ΔHBS} and FGF2^{ΔHBS}. In very specific embodiments the modified FGF consists of FGF 1^{ΔHBS} FGF2^{ΔHBS}, or combinations thereof and heparin is not administered with the modified FGF.

The following Examples are set forth to support certain scientific tenets and to illustrate specific aspects and embodiments and should not be construed as limiting the full scope of the invention as defined by the appended claims.

### EXAMPLES

### Example 1

The series of experiments constituting this example explore the effects of heparin on the cardioprotective effects of FGF1, suggest a mechanism that guides development of modified FGF engineered to reduce heparin-binding affinity, and affirms the efficacy of the therapeutic in reducing reperfusion injury in the presence of heparin.

Taken together, the findings set forth in the EXAMPLES demonstrate that: 1) intravenous administration of FGF1 at the onset of reperfusion protects the heart against cardiac ischemia injury, while heparin reduces the protective effect of FGF1; 2) heparin reduces the availability of FGF1 to the heart, which may be potential mechanism(s) of why heparin lessens the cardioprotective effect of FGF1, 3) a modified FGF1 ligand with reduced heparin binding affinity (FGF1^{ΔHBS}) lowers infarct size and improves cardiac function in the presence of heparin, although it has a similar elimination half-life profile of native FGF1, and 4) FGF1^{ΔHBS} enhances the cardioprotective signaling (e.g., ERK activation) even in the presence of heparin to a greater extent than native FGF1.

Overview of Methods and Results: A rat model of MI was employed to compare cardioprotective potentials (lower infarct size and improve post-ischemic function) of native FGF1 and an engineered FGF1 (FGF1^{ΔHBS}) with reduced heparin-binding affinity when given at the onset of reperfusion in the absence or presence of heparin. FGF1 and FGF 1^{ΔHBS} did not alter heparin's anticoagulant properties. Treatment with heparin alone or native FGF1 significantly reduced infarct size compared to saline (p<0.05). Surprisingly, treatment with FGF 1^{ΔHBS} markedly lowered infarct size compared to FGF1 (p<0.05). Both native and modified FGF1 restored contractile and relaxation function (p<0.05 versus saline or heparin). Furthermore, FGF 1^{ΔHBS} had greater improvement in cardiac function compared to FGF1 (p<0.05). Heparin negatively impacted the cardioprotective effects (infarct size, post-ischemic recovery of function) of FGF1 (p<0.05) but not of FGF1^{ΔHBS}. Heparin also reduced the biodistribution of FGF1, but not FGF1^{ΔHBS}, to the left ventricle. FGF1 and FGF 1^{ΔHBS} bound and triggered FGFR1-induced downstream activation of ERK1/2 (p<0.05); yet, heparin co-treatment decreased FGF1-produced ERK1/2 activation, but not that activated by FGF1^{ΔHBS}. Conclusion: These findings demonstrate that modification of the heparin-binding region of FGF1 significantly improves the cardioprotective efficacy, even in the presence of heparin, identifying a novel FGF ligand for therapeutic use in ischemic heart disease.

### Pharmacological Agents

Heparin sodium (1000U/mL) was purchased from Sigma-Aldrich (MO, USA). Recombinant human hexa-histidine-tagged FGF1 (His-FGF1) were obtained from Dr. Moosa Mohammadi's laboratory. Non-tagged FGF1 was obtained from R&D system (USA). The
FGF1^{ΔHBS} mutant, which carries the K127D/K128Q/K133V triple mutation in its HS-binding region (**Fig. 1A**) was expressed in E.coli and purified to homogeneity using anion exchange chromatography followed by size exclusion chromatography (**Fig. 1B**) in Dr. M. Mohammadi's lab. Heparin and FGFs were diluted in phosphate-buffered saline immediately before use.

### Animals

Male Sprague Dawley rats weighing 240-300g supplied by Harlan Laboratories were housed and handled according to the standards and guidelines set by the Care and Use of Laboratory Animals published by the US NIH (NIH Publication No. 85-23, revised 2011). All animal experimental protocols were approved by the University of Cincinnati Institutional Animal Care and Use Committee. All animals were acclimatized for at least 48 hours before experimental use.

### Experimental protocol and exclusion criteria

Rats were randomly divided into four sets of studies. Two sets were to assess the pharmacodynamics effects cardioprotective efficacy of native FGF1 and FGF 1^{ΔHBS} and FGF1
CVR-2016-799R1 signaling and the other two sets were to evaluate the pharmacokinetic properties of native FGF1 and modified FGF1 (FGF1^{ΔHBS}). The first set of experiments was for the ischemia/reperfusion study in which animals were divided into six groups as follows: (1) saline group: saline treatment given intravenously for 10 minutes immediately upon reperfusion, (2) heparin group: heparin, 60 U/kg intravenous (i.v.) bolus, given immediately at reperfusion + 12 U/kg/hr i.v. infusion for 120 minutes of reperfusion, (3) FGF1 group: FGF1, 10 µg/kg i.v. infusion, given for 10 minutes immediately at reperfusion, (4) FGF1 + heparin: heparin, 60 U/kg bolus, given immediately upon reperfusion followed by FGF1, 10 µg/kg i.v. infusion, given for 10 minutes and 12 U/kg/hr i.v. infusion given for 110 minutes of reperfusion, (5) modified FGF1 (FGF1^{ΔHBS}) group: FGF1^{ΔHBS}, 10 µg/kg i.v. infusion, given for 10 minutes, or (6) FGF 1^{ΔHBS} + heparin: heparin, 60 U/kg bolus, given immediately at reperfusion followed by FGF 1^{ΔHBS}, 10 µg/kg i.v. infusion, given for 10 minutes and 12 U/kg/hr i.v. infusion for 110 minutes of reperfusion. All rats were subjected to 30 minutes of regional ischemia and 120 minutes of reperfusion. Drugs were administered at the onset of reperfusion via the jugular vein (**Fig. 2A**). The final dose (10 µg/kg, i.v.) of native and modified FGF1 was determined by performing preliminary dose response studies (1 µg/kg, 10 µg/kg, and 100 µg/kg; data not shown); the dose range was derived from published experiments (2.6 µg/kg, i.v. and 10 µg/kg, i.v.) that have previously been shown to be effective in protecting the heart against ischemia-reperfusion injury in rodent models. A total of ninety-six rats was completed for the I/R study. Sample size for each group ranged from 9-16 depending on the power analysis. A total of thirty-five rats were excluded from the study based on the lack of cyanosis of the heart or typical elevation of the ST segment in the electrocardiogram during ischemia, and death from malignant arrhythmia. For the biodistribution or FGF1 signaling study, rats were randomly divided into six groups as described above (**Fig. 2B**). Following drug treatment (immediately following 15 minutes of treatment or 105 minutes post-treatment at the point which mimics the length of time of reperfusion), solid organs and blood (see sections "Tissue collection for exogenous FGF1 biodistribution" and "Cardiac preparation to detect the activation of FGF1 downstream molecules") were collected. A total of 33 rats were used for biodistribution and FGF1 signaling studies, and 3 rats were excluded due to anesthetic overdose. Also, a total of 8 plasma samples were excluded because of hemolysis. For elimination half-life of native FGF1 and FGF1^{ΔHBS}, 12 rats were randomly divided into two groups and treated intraperitoneally with either FGF1 form as described in the section "Pharmacokinetic evaluation of exogenous FGF1 or FGF1^{ΔHBS}".

### Assessment of blood clotting time

In sets of experiments, blood clotting time was detected at baseline and 15 minutes after drug treatment. In brief, a nick 1-2mm depth was made at 5 cm and 3 cm of the proximal tail for baseline and post-drug blood clotting time, respectively; the tail nick was blotted with 4x4 gauze every 15 seconds until bleeding totally stopped, and blood clotting time was recorded from the onset of bleeding until it stopped.

### In Vivo I/R model

The *in vivo* I/R model was established by ligating and loosening the left anterior CVR-2016-799R1 descending artery (LAD) previously described by our laboratory. In brief, rats were anesthetized with thiobutabarbital sodium salt hydrate, Inactin^{®} hydrate (100 mg/kg, i.p., Sigma-Aldrich), and intubated and artificially ventilated via a rodent respirator (respiratory rate: 75 strokes/minute; tidal volume: 3 mL; Model 683, Harvard, USA). The heart was exposed through a left thoracotomy and pericardiotomy, and a 6-0 silk suture was placed around the left anterior descending (LAD) coronary artery. After equilibration for 15 minutes, hearts were subjected to 30 minutes of ischemia by ligating the LAD via a snare occluder and reperfusion occurred for 120 minute via loosening the snare occluder. The appearance of cyanosis of the heart and significant ST-segment elevation in the electrocardiogram were used to verify successful ligation.

### Hemodynamic analysis

Hemodynamic measurement was assessed via a Millar MIKRO-TIP transducer (SPR-320, Houston, TX, USA) placed into the left ventricle via the right carotid artery. Left ventricular systolic (LVSP), end-diastolic pressure (LVEDP), heart rate (HR), rate of contraction (+dP/dt) as well as rate of relaxation (-dP/dt) were collected (Digi-Med data analysis) during specific timepoints of baseline, ischemia and reperfusion (**Fig. 2A**).

### Infarct size measurement

At the end of the ischemia-reperfusion injury study, the LAD was re-occluded with the suture used previously for the establishment of ischemia. 5% Evans blue dye solution was given via the jugular vein to define the area-at-risk (AAR). Then, the heart was arrested in diastole with 1 mL of 15% potassium chloride, and the animal was euthanized with an anesthetic overdose followed by a bilateral pneumothorax for removal of the heart. The heart was excised, washed with saline, frozen in -80oC for 5-6 minutes, sliced transversely into 2-3 mm thick sections, incubated in 1% 2,3,5-triphenyl tetrazolium chloride (TTC, pH 7.4) for 30 minutes at 37°C in the dark followed by fixing in 4% paraformaldehyde and digitally photographed. Infarct size (IS), depicted as the percentage of the area-at-risk, was determined by Image J software (NIH, 1.61 version).

### Pharmacokinetic evaluation of exogenous FGF1 or FGF1^{ΔHBS}

The *in vivo* half-life of FGF1 or FGF 1^{ΔHBS} (given as a single, intraperitoneal dose of 0.5 mg/kg body weight) was assessed, using the human FGF1 immunoassay ELISA kit (R&D System, MN), from blood (i.e., serum) collected at varying times as described previously. The pharmacokinetic parameters of FGF1 or FGF 1^{ΔHBS} were analyzed using the Drug and Statistics Software (DAS, v 2.0; Mathematical Pharmacology Professional Committee of China).

### Tissue collection for exogenous FGF1 biodistribution

The rat was anesthetized with Inactin^{®} hydrate (100 mg/kg, i.p.), and the external jugular vein was isolated for saline, FGF1, FGF 1^{ΔHBS} (mutant FGF1) and/or heparin administration. At the end of the experiment (based on half-life of FGF), the animal was euthanized with an anesthetic overdose followed by excision and collection of the heart (including atria, left ventricle, and right ventricle), liver, lungs, kidneys, spleen, skeletal muscle, and brain (negative control as i.v. administered FGFs do not cross the blood-brain barrier). Blood from the jugular vein was collected into the tubes containing 0.5M EDTA and complete mini protease inhibitor cocktail (Roche), centrifuged at 2,000 g for 15 minutes at 4° C and the separated plasma was saved. Both tissue and plasma were snap-frozen in liquid nitrogen and stored at -80° C for evaluation of exogenous FGF 1 biodistribution.

### Enzyme-linked Immunosorbent Assays (ELISA) of exogenous FGF1

Snap-frozen tissue samples were powdered and homogenized in protein extraction buffer (20 mM Tris, 2 mM EDTA, 2 M NaCl, 1% NP40 with Roche complete mini EDTA-free protease inhibitor cocktail (1 tablet/10 mL) and 1 mM PMSF added right before use) and protein was extracted as previously described. Exogenous levels of tissue and plasma FGF1 and FGF 1^{ΔHBS} were determined by ELISA per the manufacturer's instructions (FGF1 immunoassay kit, R&D Systems). In brief, 80 µg protein was incubated with assay diluent for 2 hours at room temperature, shaking in a 96-well plate coated with a monoclonal antibody against FGF1. After 4 washes, conjugate buffer was added followed by incubation for 2 hours at ambient temperature. All samples were incubated for 30 minutes in the dark with substrate solution, followed by stop solution. The optical density (O.D) was measured at 450 nm with correction wavelength of 570 nm in GENios Microplate Reader (Tecan, USA). The FGF1 standard in the kit was diluted with assay diluent to produce a dilution series of 2000 pg/mL, 1000 pg/mL, 500 pg/mL, 250 pg/mL, 125 pg/mL, 62.5 pg/mL, and 31.2 pg/mL. The assay diluent alone served as the zero standard (0 pg/mL). Standard and unknown samples were assayed in duplicate. Standard curve was created by using Excel via plotting the log of the average FGF1 concentrations versus the log of the O.D. The concentration of exogenous FGF1 (native or mutant) in tissue and plasma samples was calculated from the standard curve.

### Cardiac preparation to detect the activation of FGF1 downstream molecules

Hearts from rats treated with saline, native FGF1 or FGF 1^{ΔHBS} in the presence or absence of heparin were collected immediately after administration and at 120 minutes post-treatment for evaluation of downstream signaling. One-half of each snap-frozen heart was powdered and homogenized in protein extraction buffer as previously described by our laboratory. For FGFR1, 150 µg of total protein were loaded onto a 8% SDS-PAGE gel, for ERK1/2 activation, 20 µg of whole cell protein homogenate was loaded onto a 15% SDS-PAGE gel, and for PKCα and PKCδ, 100 µg of whole cell protein homogenate was loaded onto a 10% SDS-PAGE gel and then transferred to nitrocellulose membrane. 0.1% Ponceau S in 5% acetic acid was used to examine the transfer efficiency and loading equality as described previously43. Membranes were blocked with 5% dry milk and then incubated with primary antibody against phospho-FGFR1 (Y-654, 1:500, Cell Signaling), phospho-ERK1/2 (1: 1000, Cell Signaling) or phospho-PKCα (1:500, Santa Cruz) or phospho-PKCδ (1:500, Santa Cruz) followed by incubation with HRP-conjugated secondary goat anti-rabbit antibody (1:3000, Santa Cruz Biotechnology). Membranes were stripped and reprobed with antibodies against total FGFR1 (1:500, Cell Signaling), total ERK (1:1000, BD Transduction Labs) or total PKCα (1:500, Santa Cruz) or PKCδ (1:500, Santa Cruz). Bound antibodies were visualized by ECL system. Activation is indicated as a ratio of phosphorylated protein/total protein. β-actin (1:1000, Cell Signaling) was also used as a loading control.

To detect levels of phospho-Akt, Akt, phospho-STAT3, STAT3, and GAPDH, an automated capillary Western blot was performed according to the WES user guide by ProteinSimple^{™} and all Wes reagents were purchased with the Wes Size Separation Master Kit (ProteinSimple^{™}, San Jose, CA). The cardiac samples (collected immediately post-treatment or 110 minutes post-treatment) were mixed together with 5 × Master Mix (DTT, fluorescence labeled maker, SDS) in a ratio of 5:1 and then incubated at 70 °C for 5 min. The cardiac samples (0.4 mg/mL) and the biotin-labeled protein ladder (12 kDa, 40 kDa, 66 kDa, 116 kDa, 180 kDa and 230 kDa) were loaded into individual wells of the sample plate. Primary antibody against phospho-Akt (1:50, Cell Signaling), Akt (1:50, Cell Signaling), phospho-STAT3 (1:50, Santa Cruz), STAT3 (1:50, Millipore), and GAPDH (1:50, Cell Signaling) were diluted with antibody diluent buffer. Loading conditions were determined by GAPDH. The experiment was then performed in Wes instrument as described by Wang and colleagues.

### Statistical analysis

Data were expressed as mean ± standard error of the mean (SEM). Differences between groups were assessed by one-way or two-way ANOVA with Student-Newman-Keul post-hoc test or Kruskal-Wallace non-parametric test with Tukey's post-hoc test, depending on the endpoint evaluated. P<0.05 was considered statistically significant.

### Results

### FGF1 given immediately at reperfusion protects the heart against ischemia-reperfusion injury.

Previous studies reported that pre-treatment with FGF1 prior to an ischemic insult improved cardiac function and enhanced cardiomyocyte survival. As these studies indicate that this growth factor intervention protects the heart only when given prior to an ischemic event, its utility for use in the clinical setting of acute MI is limited. Therefore, elucidation of (non-angiogenic) cardioprotective activity of FGF1 when administered following an acute ischemic (MI) event is a necessary step to develop FGF1 as a potential therapeutic agent. Consistent with Cuevas and investigators, our results demonstrate that human recombinant FGF1 given immediately at the onset of reperfusion rescued cardiomyocyte survival and cardiac function as represented by a reduction in infarct size and an improvement in post-ischemic contractility and relaxation (±dP/dt), respectively (p<0.05, **Figs. 3A-3B** and **Figs. 4A-4F****, Table 1,** **Fig. 9**).

### Heparin co-therapy reduces the cardioprotective effect of FGF1

Heparin treatment, either as unfractionated or low molecular weight (enoxaparin), is a standard medical practice for patients with acute MI. Since FGF1 is a heparin-binding protein, issues of heparin co-medication (per the 2010 NHLBI Workshop recommendations) must be taken into consideration when developing FGF1 for acute MI therapy. To determine the effect of heparin on the cardioprotective activity of FGF1, infarct size and cardiac function was evaluated in the absence and presence of heparin co-administration (**Fig. 2A**). Compared to saline treatment (58% infarct), heparin alone, at reperfusion, reduced infarct size (46% infarct), but not to the degree of FGF1 treatment (35% infarct, **Fig. 3B****,** p<0.05); the heparin-induced reduction of myocardial infarct was similar to that first reported by Dr. Lucchesi's group in the 1990s. Notably, heparin co-administration significantly inhibited FGF 1-induced cardioprotection against MI (42% infarct, **Fig. 3B****,** p<0.05). In addition, rats treated with FGF1 recovered to 69% of its baseline contractility compared to saline treatment (44%, **Figs 4A** and **4E****, Table 1,** **Fig. 9** p<0.05). After 2 hours of reperfusion, there was a significant difference in post-ischemic contractility between FGF1 treatment (69%) versus FGF1+heparin (57%) co-therapy (**Figs. 4A** and **4E****, Table 1,** **Fig. 9** p<0.05), suggesting that heparin attenuates the FGF1-induced protection against post-ischemic cardiac dysfunction. There was no significant difference in post-ischemic contractility between the saline and heparin group (**Fig. 4A** and **4E****, Table 1,** **Fig. 9** p<0.05). Other LV measures are depicted in **Table 1,** **Fig. 9** (and **Figs. 4B** and **4F**) showing that rat hearts treated with FGF1 also have improved relaxation compared to saline treatment. Taken together, these data demonstrate that heparin reduces the cardioprotective effect of FGF1 against myocardial infarction and post-ischemic recovery of cardiac function.

### A Triple mutation in the FGF1 heparin binding site (FGF1 mutant = FGF1^{ΔHBS}) is cardioprotective, leading to reduced infarct size and improved cardiac function even in the presence of heparin co-treatment compared to native FGF1.

Based on the above data demonstrating that heparin inhibits the cardioprotective effect of FGF1 (**Figs. 3A-3B** and **Fig. 4A- 4F****, Table 1,** **Fig. 9**), the question remains as to whether altering the HS-binding region of FGF1 to affect its binding affinity for heparin may change the cardioprotective efficacy of FGF1. From the crystallographic data, three basic residues at the HS binding site of FGF1, namely, K127, K128, and K133V, mediate the majority of hydrogen bonding with HS (**Fig. 1A**). Accordingly, the present investigators reasoned that mutations of these three HS-binding residues should cause a major reduction in the HS-binding affinity of FGF1. The mutated FGF1 should retain some ability to promote HS-mediated FGFR dimerization and activation. A FGF1 mutant termed FGF1^{ΔHBS}, which carries the triple mutation in its HS-binding site, was designed. FGF 1^{ΔHBS} was expressed in *E. coli* and purified to homogeneity using anion exchange chromatography followed by size exclusion chromatography. FGF1^{ΔHBS} elutes as a monodisperse peak at the predicted retention time, indicating that the mutations do not harm the tertiary folding of the ligand (**Fig. 1B**)**,** as K127, K128, and K133 are surface-exposed and do not play any role in ligand folding.

To test the impact of the mutations on HS-binding ability of FGF1, isothermal titration calorimetry (ITC) was used to compare the interactions of native FGF1 and FGF 1^{ΔHBS} with Sucrose Octasulfate (SOS), a known surrogate for HS. As shown in **Figs. 1C** and **1D****,** native FGF1 bound SOS with a Kd of 4 □M; whereas, binding of FGF1^{ΔHBS} to SOS was negligible, demonstrating that the FGF 1^{ΔHBS} mutant sustains a substantial loss in HS binding. Next, the cardioprotective potential of FGF 1^{ΔHBS} mutant when administered immediately upon reperfusion was evaluated in an *in vivo* rat model of myocardial infarction. FGF1^{ΔHBS} was ~2-fold more efficacious in reducing infarct size than native FGF1 (62% versus 39% reduction, respectively; **Fig. 3A-3B****,** p<0.05). Importantly, heparin co-therapy had minor, and no statistically significant, inhibition on the cardioprotective effect of FGF 1^{ΔHBS}; whereas, the cardioprotective activity of native FGF1 was markedly attenuated in the presence of heparin (**Fig. 3A-3B**). Of note, FGF 1^{ΔHBS} significantly reduced the infarct size to an extent that was lower than native FGF1 in the absence (22% vs 35%, respectively, p<0.05, **Fig. 3A-3B**) or presence of heparin (32% vs 42%, p<0.05, **Fig. 4A-4F**), which indicates that with the same dosage, FGF 1^{ΔHBS} was more efficacious than the native counterpart in protecting the heart from I/R injury. Both contractile and relaxation parameters were significantly improved +dP/dt and -dP/dt were markedly restored in FGF1^{ΔHBS} and FGF1^{ΔHBS} +heparin following ischemic injury (**Figs. 4C-4F****, Table 1,** **Fig. 9** p<0.05), demonstrating that the recovery of post-ischemic cardiac function is not compromised by the presence of heparin. Interestingly, the recovery of post-ischemic cardiac function of FGF 1^{ΔHBS} or FGF 1^{ΔHBS} +heparin was significantly improved compared to the native FGF1 cohorts (**Figs. 3A-3B****,** **Figs. 4A-4F****, Table 1,** **Fig. 9****,** p<0.05). There were no significant differences in heart rate with the FGF1^{ΔHBS} and FGF1^{ΔHBS} +heparin groups compared to the saline group **(Table 1,** **Fig. 9****).**

### FGF1 and FGF1^{ΔHBS} do not affect the anticoagulant activity of heparin

When developing novel agents to treat ischemic heart disease, consideration needs to be made that any new therapy does not modify and/or interfere with heparin and its anticoagulant function, which is used as the standard of care for cardiac patients, and therefore, would make the new therapy not clinically applicable. Therefore, we evaluated the effect of native FGF1 and FGF 1^{ΔHBS} mutant on the coagulation time. There was no significant difference in blood clotting time during baseline (pre-treatment) or following administration of saline, FGF1 or FGF1^{ΔHBS} (post-treatment). However, in the heparin, FGF1+heparin and FGF 1^{ΔHBS} +heparin groups, the coagulation time was significantly longer post-treatment than at baseline or following administration of saline, FGF1 or FGF 1^{ΔHBS} **(Table 2,** **Fig. 10****)**. These data demonstrate that neither native nor HS-binding mutant FGF1 co-administered with unfractionated heparin co-treatment affect the anticoagulant property of heparin therapy alone.

### FGF1 and modified FGF1 (FGF1^{ΔHBS}) have similar elimination half-life.

One possibility to account for the enhanced cardioprotective activity of FGF1^{ΔHBS} was that the elimination half-life was varied from native FGF1. Mean serum concentration-time profiles for FGF1 and FGF1^{ΔHBS} are shown in **Table 3,** **Fig. 11****.** The non-compartmental *in vivo* pharmacokinetic properties of FGF1 and FGF 1^{ΔHBS} showed that the absorption phase half-life (t1/2z) and the elimination phase half-life (t1/2z) of FGF 1^{ΔHBS} were not significantly different from FGF1. The maximum serum concentration (Cmax) and area-under-curve (AUC) for FGF 1^{ΔHBS} were 4-fold and 2-fold greater than those for FGF1, respectively. However, the mean time to reach maximum serum concentration (Tmax) of FGF 1^{ΔHBS} was reached earlier than with FGF1. This result implies that the absorption rate of FGF 1^{ΔHBS} after intraperitoneal administration was relatively fast, which is to be expected as the weak HS binding affinity of FGF 1^{ΔHBS} should allow this ligand to avoid entrapment by HS in the pericellular/extracellular milieu.

### Heparin co-treatment modifies the biodistribution of exogenous FGF1 to the heart

It is reported that heparan sulfates on the cell surface or extracellular matrix can concentrate or sequester FGFs which accounts for some of the modulatory activity of heparin on FGFs. Therefore, the effect of heparin on exogenous FGF1 tissue localization was determined. Saline treatment is indicative of the endogenous level of FGF1 for each organ or in plasma. There was no difference in saline or heparin treatment with regard to FGF1 levels in plasma or organs evaluated. This suggests that heparin treatment does not lead to secretion of endogenous FGF1 from the organs. Compared to saline treatment, a significant amount of exogenous FGF1 was distributed to kidney, spleen, heart, liver, and plasma, while less went to lung and skeletal muscle (**Figs. 5A** and **5C****,** p<0.05). However, upon co-administration of FGF1 and heparin, exogenous FGF1 biodistribution was significantly decreased in kidney, spleen, heart, liver and plasma **(****Figs. 5A** and **5D****,** p<0.05), suggesting heparin could change the biodistribution of exogenous FGF1 and influence the cardioprotective efficacy of FGF1. Most notably, when in the presence of heparin therapy, the greatest reduction in FGF1 biodistribution was at the heart **(****Fig. 5A** and **5D****,** p<0.05). These findings are consistent with other observations that heparin modifies the distribution of FGF1 treatment. The next question is whether heparin alters the biodistribution of FGF 1^{ΔHBS}. As shown in **Fig. 5B** and **Fig. 5C****,** in the absence of heparin, FGF 1^{ΔHBS} mainly went to the heart including atria, left ventricle, and right ventricle. In fact, there was a greater biodistribution of FGF 1^{ΔHBS} to the left ventricle compared to native FGF 1. In the presence of heparin, the amount of FGF 1^{ΔHBS} to the heart was similar to that of FGF 1^{ΔHBS} alone (**Fig. 5B** and **Fig. 5D**). These findings indicate that modifying the heparin-binding region of FGF1 retains and, even, enhances its biodistribution/targeting to the heart even in the presence of heparin therapy, and demonstrates that heparin can no longer sequester FGF1 from its target site, the heart.

### Heparin co-therapy does not alter the activation of FGF1 downstream signaling triggered by modified FGF1 (FGF1^{Δ}^{HBS})

To ensure that the mutations in the heparin-binding site did not impair FGF binding to its receptor, ITC was used to compare the binding interactions of native FGF 1 and FGF 1^{ΔHBS} with the extracellular ligand binding domain of FGFR3c, one of the cognate FGFRs of FGF1. Native FGF1 and FGF 1^{ΔHBS} affinities to FGFR3c ecodomain show that the HS-binding site mutations do not impact FGFR binding ability of FGF 1 (**Figs. 6A** and **6B**). Importantly, the ITC data showing that the FGF 1^{ΔHBS} retains normal receptor binding affinity confirm that the HS mutations have no adverse effect on tertiary folding of the ligand. In support of this, FGFR1 activation (i.e., tyrosine phosphorylation) *in vivo* by FGF1 or FGF1^{ΔHBS} was similar (**Fig. 6C**).

It is reported that a low concentration of heparin restored the activity of FGF1 in HS-deficient cells *in vitro,* while a high concentration of heparin completely inhibited its function. To determine the influence of heparin on the activity of FGF 1-FGFR1 signaling in the heart, ERK, PKC, Akt, and STAT3 activation, well-known pathways downstream of FGFR and RISK (reperfusion injury salvage kinase) and SAFE (survivor activating factor enhancement) mechanisms of cardioprotection, were assessed in left ventricle collected immediately after and 120 minutes post-drug administration. Immediately post-FGF 1, FGF 1+heparin, FGF 1^{ΔHBS}, or FGF 1^{ΔHBS} +heparin treatment, phosphorylation of ERK1/2 was significantly increased compared to saline or heparin only treatment (**Fig. 7A****,** p<0.05). However, heparin markedly reduced ERK1/2 activation stimulated by FGF1 (**Fig. 7A****,** p<0.05). Surprisingly, there was a significantly higher level of phosphorylated ERK 1/2 in the hearts collected from the FGF1^{ΔHBS}-treated group compared to native FGF1 treatment whether in the absence or presence of heparin (**Fig. 7A****,** p<0.05). By 2 hours post-treatment, ERK activation returned to saline control levels (**Fig. 7B****).** Although heparin treatment had a minor cardioprotective effect (**Figs. 4A-4F**), it was most likely not via ERK as its activation was not different than saline treatment (**Fig. 7A**). Neither activation of PKCα or PKCδ nor activation of Akt of the RISK pathway and STAT3 of the SAFE pathway were different among the treatment groups (**Figs. 7C-E**)**.** These data suggest that both FGF1 and FGF1^{ΔHBS} activated FGFR1 signaling (i.e., ERK1/2); yet, heparin co-therapy reduced FGF1 signaling, but not that of FGF 1^{ΔHBS}

### Example 2

This experiment compares the biodistribution of native and heparin-binding mutants FGF^{ΔHBS} recombinant human FGFs following i.v. administration; n = 84.

Targeted FGF delivery to sites of action may be regulated by heparin and HS; yet, little is known about the targeting of FGFs to the heart following disruption of their HS/heparin-binding domains. It has been shown that i.v. administration of rhFGF2 in rat results in delivery to liver, kidneys, and spleen and to a lesser extent, heart and lungs; this biodistribution was primarily due to HS proteoglycan interactions. Targeting the native and heparin-binding modified (FGF^{ΔHBS}) rhFGFs to the heart, i.e., site of cardioprotective action, is assessed using histidine (his)-tagged native and mutant FGFs via the following groups:

| | |
|---|---|
| Group I: | vehicle treatment |
| Group II: | native rhFGF1 or rh FGF1^{ΔHBS} |
| Group III: | native rhFGF2 or rhFGF2^{ΔHBS} |
| Group IV: | native rhFGF4 or rhFGF4^{ΔHBS} |
| Group V: | native rhFGF5 or rhFGF5^{ΔHBS} |

Groups 1-V are also performed with co-administration of unfractionated or low molecular weight heparin.

Construction of his-tagged native and heparin-binding modified rhFGFs as well as their expression and purification is performed as previously described using his-tagged expression vectors and nickel-nitrilotriacetic acid resin columns. Detection of the tissue distribution of native and mutated (FGF^{ΔHBS}) ligands is carried out as follows: his-tagged native and mutant FGFs (FGF1 [10 µg/kg], FGF2 [10 µg/kg], FGF4 ([1 µg/kg], FGF5[10 µg/kg]) are administered intravenously for 15 minutes to Sprague Dawley rats (200-225 g. 49-52 days old), and 105 minutes later (based on half-life of FGF1 and FGF2, to provide a time point analogous to 120 minutes reperfusion in FGF-treated IR studies, the heart, liver, lungs, kidney, spleen, skeletal muscle, plasma, and brain (negative control as i.v. administered FGFs do not cross the blood-brain barrier) are harvested for analysis of their tissue distribution. His-tagged FGFs are detected in solid organs by ELISA (**Fig. 14**) both for FGF and histidine (data not shown), and immunoprecipitation (IP) followed by immunoblot and in plasma by ELISA as described previously. His-tags are widely used because they are small and rarely interfere with function, activity, or structure of target proteins. To ensure that the histidine tag does not interfere with FGF activity, immunoblots are performed to evaluate whether downstream FGF signaling (e.g., ERK) and FGF receptor activation occurred to a similar degree as untagged FGF treatment. **Fig. 19** demonstrates that ERK activation (phosphor-ERK) as well as FGFR1 activation (phospho-FGFR1) is similarly and markedly increased irrespective of whether rats were treated with FGF1 labeled with a histidine tag (lane 2) or unlabeled FGF1 (lane 3) compared to saline treatment (lane 1). There is no observed difference in total ERK or FGFR1 expression between the treatment groups. This finding suggests that his-tagged FGF does not interfere with FGF signaling as anticipated.

There is evidence that some FGFs, such as FGF4, which normally do not have effects on a particular tissue or cell type, have biological activity at that target site in the presence of heparin. Therefore, tissue distribution (heart, lung, liver, kidney, spleen and brain) of his-tagged native and mutant (FGF^{ΔHBS}) FGFs in the presence of heparin (unfractionated heparin: 12 units/kg/hr, intravenously or low molecular weight heparin [enoxaparin]: 1 mg/kg, subcutaneously), is determined. These heparins are most commonly used clinically in acute MI and heparin doses chosen are based on standard human clinical dosing. Tissue preparation and analysis is performed as described above.

Data (**Fig. 14**) demonstrate that exogenous treatment of FGF2 leads to an increased biodistribution heart (left ventricle). Upon heparin co-administration, exogenous FGF2 is no longer targeted to the heart; excitingly, this provides a potential mechanism for the observation (**Fig. 13**) of reduced FGF2 efficacy in lowering infarct size when it is given in the presence of heparin (46% infarct) versus when FGF2 is given alone (26% infarct, p<0.05).

### Example 3

This experiment demonstrates bioavailability of native and thermally stable mutants (FGF^{ΔTS}) rhFGFs following intravenous administration; (n=102 rats).

Ligand bioavailability (i.e., pharmacokinetic mechanism) can be a significant pharmacological limitation for therapeutic applicability. *In vitro* data previously generated indicates that enhancing the protein stability of FGF 1 increased its half-life (**Fig. 16**). Susceptibility to protease action of FGF1^{ΔTS} was determined by monitoring changes in fluorescence emission during degradation by trypsin, FGF1^{ΔTS} (20-fold increase) and FGF2 (7-fold increase) were more resistant to proteolytic degradation than the native FGF 1. It remained to be elucidated as to whether these pharmacokinetic mechanisms I.e., bioavailability and/or half-life) were enhanced *in vivo* as well. Therefore, detection of the bioavailability and half-life of native and thermally stable mutated ligands (FGF^{ΔTS}) are carried out as follows:

| | |
|---|---|
| Group I: | vehicle treatment |
| Group II: | native rhFGF1 or rhFGF^{1ΔTS} |
| Group III: | native rhFGF2 or rhFGF2^{ΔTS} |
| Group IV: | native rhFGF4 or rhFGF4^{ΔTS} |
| Group V: | native rhFGF5 or rhFGF5^{ΔTS} |

Groups 1-V are also performed with co-administration of unfractionated or low molecular weight heparin and the following doses are employed: (FGF1 [1 µg/kg and 10 µg/kg], FGF2 ([1 µg/kg and 10 µg/kg], FGF4[0.1 µg/kg and 1 µg/kg), FGF5 (1 µg/kg and 10 µg/kg]) and are administered intravenously for 15 minutes to Sprague Dawley rats (200-225 g. 49-52 days old). Based on published data on the half-life of native FGF1, FGF2, FGF4, and FGF5, serial blood collection (50µL) occurs every 5 minutes following growth factor infusion up to 105 minutes later (to provide a time point analogous to 120 minutes reperfusion in FGF-treated IR studies) and plasma is isolated for FGF levels via ELISA. As previously described for heparin-binding growth factor - 1, a semilog plot is constructed to represent the blood concentration of the FGF as a function of time and extrapolated back to time zero. Half-life is determined from this plot as the time required for the FGF concentration to fall to half the concentration at time zero. The plot resembled the standard clearance curve of a drug following first-order kinetics, which is confirmed by the linearity of the semilog plot of these data. The clearance curve is divided into an initial distribution phase followed by a terminal elimination phase. Elimination or biological half-life is determined directly from the semilog plot.

There is evidence that FGF half-life is altered when heparin is co-administered. Therefore, half-life of native and thermally stable mutant (FGF^{ΔTS}) FGFs in the presence of clinically-applied heparins (unfractionated heparin: 12 units/kg/hr, intravenously or low molecular weight heparin (enoxaparin]: 1 mg/kg, subcutaneously) 24 is also determined in the same way. These experiments provide information as to the effects of heparin co-treatment on FGF^{ΔTS}s' half-life and bioavailability to the heart.

### Example 4

These experiments compare the biodistribution and bioavailability of native and mutant rhFGFs with less heparin binding and greater protein stability following intravenous administration; (n=102 rats).

FGF^{ΔHBS/ΔTS} mutants are constructed (for exemplary design of a specific FGF1 ΔTS mutant see J. Biol. Chem.; 2009; 284:25388-25403; the present inventors designed novel mutants possessing different mutations and exhibiting increased thermal stability for FGF2, FGF4 and FGF5). Besides the protein stabilizing point mutations, these variants carry mutations within the HS binding domain, resulting in reduced heparin binding. FGF mutants are readily produced in E. coli and purified to homogeneity using affinity chromatography followed by size exclusion chromatography. Fluorescence and circular dichroism measures confirm proper folding of the mutants and competitive binding experiment in NIH3T3 cells with radiolabeled native FGF verify that the introduced mutations did not impair growth factor binding to the receptor.

Pharmacokinetic properties of FGF^{ΔHBS/ΔTS} mutants are assessed in the absence or presence of clinically-applied heparin formulations. By manipulating HS binding properties of FGFs, ligands are developed that exhibit more biodistribution to the heart. For example, FGF2 recognizes heparan sulfates (HSs) ubiquitously; whereas, FGF4 fails to bind cardiac- and vascular-expressed HSs and become active. Yet, administering heparin with FGF4 leads to binding of vascular-expressed heparan sulfates and angiogenesis. Conversely, Hondermack et al. (Experientia. 1990;46:973-974) demonstrated that simultaneous injection of FGFs with heparin weakened FGF binding to vessels in a dose-dependent manner. Moreover, clearance of FGF2 in humans or FGF1 in experimental models has been shown to be decreased. This suggests that heparin/FGF complexes may alter biological responses. Based on published *in vitro* data, showing that enhancing the protein stability of FGF1 increased its half-life (**Fig. 16**), modifications were designed to increase bioavailability and half-life *in vivo,* thereby more FGF is targeted to the heart and lower doses of the thermally-stable modified FGFs (FGF^{ΔTS}) are required for cardioprotection.

The following several examples show that Heparin co-therapy diminishes the cardioprotective effects of native FGF, but not of the modified FGF.

### Example 5

Beneficial effects of FGFs (such as FGF1, 2, 4, and 5) in cardiomyocyte survival and/or angiogenesis during IR injury have been demonstrated. Yet, there is limited understanding of the efficacy of these molecules against acute MI when administered at reperfusion. Data herein shows that FGF2 administered immediately at reperfusion protects the rodent heart against cardiac dysfunction and MI (**Figs. 12A, 12B** and **Fig. 13**). Heparin treatment, either as unfractionated or low molecular weight (enoxaparin), is a standard medical practice for patients with acute MI. Since FGFs are heparin-binding proteins, issues of heparin co-medication (per the 2010 NHLBI Workshop recommendations) must be taken into consideration when developing FGFs for acute MI therapy.

Identifying FGFs necessary for cardioprotection against post-ischemic cardiac dysfunction and myocardial infarction is addressed using an *in vivo* rat model of acute MI as previously described laboratory. Age-matched (200-225 g, 49-52 days old) Sprague Dawley rats are subjected to regional IR injury. In the rat model of acute MI, left ventricular (LV) pressure and function is assessed via a Millar MIKRO-TIP transducer placed in the LV via the right carotid artery, blood pressure (BP) measured via the femoral arty and vehicle (saline or heparin) or rhFGF treatment administered via the femoral vein. Surface ECG leads are placed to provide continuous electrocardiographic data. A left thoracotomy is performed, and regional IR injury is induced by occlusion (via 8-0 suture and snare tubing) of the left anterior descending (LAD) coronary artery for 30 minutes (ischemia) followed by a 2 hour reperfusion (initiated by release of the ligature around LAD). Systolic and diastolic BP and LV function along with post-ischemic functional recovery, a cardioprotective endpoint, as measured by positive (+) and negative (-) dP/dt as well as LV developed pressure is assessed at baseline, ischemia, and reperfusion (**Fig. 12A**). Following the IR protocol, infarct size, another cardioprotective index, are measured by histochemical staining with an indicator (1% 2,3,5-triphenyl-tetrazolium chloride, TTC) for viable vs. non-viable tissue. The LAD is re-occluded and Evans blue dye is injected intravenously to stain the normal area of LV. The heart is cut into 5-7 transverse slices for visualization of the normal, non-ischemic area and the area-at-risk (i.e., ischemic zone). These tissue areas are incubated in TTC (37°C, pH=7.4). The normal, ischemic risk and infarct areas are photographed and determined by computer morphometry, and infarct size is depicted as a percent of area at risk.

To decipher the role of individual FGFs, native rhFGF1 (10 µg/kg), rhFGF2 (10 µg/kg), rhFGF4 (1 µg/kg), rhFGF5 (10 µg/kg), or vehicle (control) is infused intravenously during the first 15 minutes of reperfusion:

| | |
|---|---|
| Group I: | 30 min Isch + 2 hrs Rep - vehicle treatment immediately at Rep for 15 min |
| Group II: | 30 min Isch + 2 hrs Rep - native rhFGF1 immediately at Rep for 15 min |
| Group III: | 30 min Isch + 2 hrs Rep - native rhFGF2 immediately at Rep for 15 min |
| Group IV: | 30 min Isch + 2 hrs Rep - native rhFGF4 immediately at Rep for 15 min |
| Group V: | 30 min Isch + 2 hrs Rep - native rhFGF5 immediately at Rep for 15 min |
| Group VI: | 30 min Isch + 2 hrs Rep - unfractionated or lmw heparin during Rep |

Groups II - V are also performed with co-administration of unfractionated or low molecular weight heparin at Rep.

Doses were determined by preliminary dose response studies derived from published experiments that have previously been shown to be effective in protecting the heart against IR injury or to produce cardiac angiogenesis in rodent or canine models. Post-ischemic recovery of cardiac function and infarct size are assessed to determine which of the rhFGFs elicit cardioprotection.

Heparan sulfate proteoglycans or heparin regulates the bioavailability of FGFs. In order to develop a "cardioprotective" FGF as a therapeutic against ischemic heart disease, it was necessary to elucidate the efficacy of these cardiac FGFs in the presence of heparin treatment. Therefore, native rhFGFs (FGF 1, 2, 4, or 5) or vehicle are infused intravenously (at dose indicated above) in conjunction with unfractionated (12 units/kg/hr, i.v.) or low molecular weight heparin (enoxaparin 1 mg.kg, s.c.) during the first 15 minutes of reperfusion (**Fig. 12A**), and post-ischemic recovery of cardiac function and the degree of myocardial infarction is assessed. Heparin formulations and doses are comparable to those administered in clinical settings. Unfractionated or low molecular weight heparin is administered alone during early reperfusion to elucidate the cardioprotective effects of heparins against IR injury. Whether FGF administration alters heparin activity is evaluated. To assess this potential interaction, activated clotting time (ACT) is assessed in vehicle-treated or FGF-treated groups in the absence or presence of heparin co-administration. Baseline and 15 minute post-drug treatment ACT is determined. It has been established that a heparin response occurs within 1 minute after an intravenous dose in humans and that no more time is required to achieve a predicted ACT. Data demonstrate that FGF (native or mutant) and unfractionated heparin co-treatment do not affect the anticoagulant activity of heparin therapy alone **(Table 4).**

**Table 4**

| Clotting time of heparin therapy in the absence or presence of FGF |
|---|
| Baseline: 161 +/- 16 sec (n = 16) |
| 15 mins post-heparin treatment: 737 +/- 127 sec (n = 4) |
| 15 mins post rhFGF2 treatment: 192 +/- 120 sec (n = 5) |
| 15 mins post rhFGF2 + hep treatment: 878 +/-305 sec (n = 4) |
| 15 mins post rhFGF1 treatment: 144 +/- 21 sec (n=7) |
| 15 mins post rhFGF1 + hep treatment: 624 +/- 100 sec (n = 5) |
| 15 mins post rhFGF^{ΔHBS} treatment: 169 +/- 10 sec (n=10) |
| 15 mins post rhFGF^{ΔHBS} + hep treatment: 540 +/- 110 sec (n=10) |

### Example 6

This experiment shows the effect of mutagenesis in the FGF heparin-binding region on cardioprotection in the absence and presence of unfractionated or low molecular weight heparin (n=180 rats).

Data demonstrate that rhFGF2 given at reperfusion using an in vivo rat model of MI protects the heart against IR injury (e.g. **Fig. 12**); however, FGF2's cardioprotective effect is inhibited in the presence of heparin therapy (**Fig. 13**). Whether altering the heparin-binding domain of FGFs will enhance or abrogate their cardioprotective activity must also be determined. Modified FGFs, in which regions of the heparin-binding domain are mutated (FGF^{ΔHBS}), were developed as shown in Example 1. FGF^{ΔHBS} are given at early reperfusion (**Fig. 12A**), at the same doses as native FGFs in the absence or presence of unfractionated or low molecular weight heparin as indicated below:

| | |
|---|---|
| Group I: | 30 min Isch + 2 hrs Rep - vehicle treatment immediately at Rep for 15 min |
| Group II: | 30 min Isch + 2 hrs Rep - rhFGF 1^{ΔHBS} immediately at Rep for 15 min |
| Group III: | 30 min Isch + 2 hrs Rep - rhFGF2^{ΔHBS} immediately at Rep for 15 min |
| Group IV: | 30 min Isch + 2 hrs Rep - rhFGF4^{ΔHBS} immediately at Rep for 15 min |
| Group V: | 30 min Isch + 2 hrs Rep - rhFGF5^{ΔHBS} immediately at Rep for 15 min |
| Group VI: | 30 min Isch + 2 hrs Rep - unfractionated or lmw heparin during Rep |

Groups II-V are also performed with co-administration of unfractionated or low molecular weight heparin at Rep; and a subset of rats are treated with native FGF1, FGF2, FGF4, FGF5 immediately at reperfusion without or with heparin formulations.

Post-ischemic recovery of cardiac function and myocardial infract size is determined to identify which mutated heparin-binding FGFs (FGF^{ΔHBS}) elicit cardioprotection, in absence or presence of heparin. ACT is used to evaluate whether mutated FGFs (FGF^{ΔHBS}) affect the anticoagulant activity of heparin. The cardioprotective activity of FGF 1^{ΔHBS} (mutated FGF1) is compared with native FGF1 in an in vivo rat model of myocardial infarction. Data (**Fig. 3B**) show that the FGF1^{ΔHBS} mutant is 2-fold more efficacious in reducing infarct size than native FGF1 (60% versus 31%). Importantly, heparin co-therapy did not reduce the cardioprotective effect of the FGF 1^{ΔHBS}; whereas, the activity of native FGF1 was lower in the presence of heparin. Area-at-risk was not different among the groups (**Fig. 3A**)**.** These new data are exciting and demonstrate the FGF^{ΔHBS} mutants as novel FGF therapeutics for acute myocardial infarction.

Whether the enhanced cardioprotective activity of FGF1^{ΔHBS} was because the elimination half-life was varied from native FGF1 was also assessed. Pharmacokinetic (PK) profiles for FGF1 and FGF 1^{ΔHBS} are shown in **Fig. 11****.** The non-compartmental *in vivo* PK properties of FGF1 and FGF 1^{ΔHBS} were not significantly different from FGF1. Mean time to reach maximum serum concentration (Tₘₐₓ) of FGF1^{ΔHBS} after intraperitoneal administration was relatively fast, which is to be expected as weak HS binding affinity of FGF 1^{ΔHBS} should allow this ligand to avoid entrapment by HS in the pericellular/extracellular milieu.

### Example 7

This experiment shows the effect of reduced heparin-binding and increased protein stability of FGFs on the cardioprotective efficacy in the absence and presence of heparin formulations, (n=210 rats).

As set forth in **Example 6,** data **(****Fig. 3B****)** demonstrate that reducing the heparin-binding of FGF1 leads to a significant decrease in infarct size in the presence of unfractionated heparin therapy compared to heparinized rats treated with the native form of FGF1. Similarly, increasing protein stability of FGF1 has a greater cardioprotective phenotype compared to native FGF1 (**Fig. 18**). Whether reducing heparin-binding as well as enhancing protein stability of FGFs results in additive cardioprotection in the presence of heparin is assessed. As described above, rats are subjected to ischemia-reperfusion injury, and native or mutant FGFs in which both heparin-binding and protein stability are altered (FGF^{ΔHBS/ΔTS}) are given immediately upon reperfusion at doses determined to be efficacious:

| | |
|---|---|
| Group I: | 30 min Isch + 2 hrs Rep - vehicle treatment immediately at Rep for 15 min |
| Group II: | 30 min Isch + 2 hrs Rep - rhFGF1^{ΔHBS/ΔTS} immediately at Rep for 15 min |
| Group III: | 30 min Isch + 2 hrs Rep - rhFGF2^{ΔHBS/ΔTS} immediately at Rep for 15 min |
| Group IV: | 30 min Isch + 2 hrs Rep - rhFGF4^{ΔHBS/ΔTS} immediately at Rep for 15 min |
| Group V: | 30 min Isch + 2 hrs Rep - rhFGF5^{ΔHBS/ΔTS} immediately at Rep for 15 min |
| Group VI: | 30 min Isch + 2 hrs Rep - unfractionated or lmw heparin during Rep |

Groups II-V are also performed with co-administration of unfractionated or low molecular weight heparin at Rep; and a subset of rats are treated with native FGF1, FGF2, FGF4, FGF5 immediately at reperfusion without or with heparin formulations (n=5).

Post-ischemic recovery of cardiac function, myocardial infarct size, and clotting time are assessed.

### Example 8

This example is a translational evaluation of the cardioprotective efficacy of mutant FGFs when co-administered at reperfusion with heparin (n=60 pigs).

Candidate FGFs are evaluated for eliciting protection against myocardial infarction and post-ischemic cardiac dysfunction in a large animal model. Novel modified FGF ligands demonstrated to have the greatest cardioprotective efficacy at the optimal dose identified in the rat model of MI are administered immediately upon reperfusion in the absence or presence of heparin formulations (based on preliminary data outcomes, the expectation is 2-3 FGFs to be studied). In the closed-chest porcine model of acute MI, LV pressure and function are assessed via echocardiography and a Millar MIKRO-TIP transducer placed in the left ventricle via the left femoral artery, BP measured via the femoral artery and vehicle (saline), heparin or rhFGF treatment administered via the right jugular vein. Surface ECG leads are placed to provide continuous electrocardiographic data. A 7F catheter is fluoroscopically guided to the left main coronary artery, and regional IR injury is induced by occlusion of the LAD coronary artery via a balloon catheter, positioned just distal to the first or second diagonal branch, for 75 minutes (ischemia) followed by 48 hours reperfusion (initiated by deflation of intracoronary balloon). Systolic and diastolic BP and LV function are assessed at baseline, ischemia, and reperfusion. Post-ischemic recovery of cardiac function, regional myocardial blood flow (via BioPAL microspheres given at 60 minutes of ischemia and 15 minutes and 48 hours of reperfusion 102 and infarct size are assessed following 48 hours or reperfusion to determine pharmacodynamic properties (efficacy and potency). Troponin samples (duplicate blood samples of 0.6 mL) are collected from the jugular catheter at 60 minutes of ischemia and 2 hours, 4 hours, 6 hours, 24 hours and 48 hours or reperfusion. Tissue is harvested and plasma is collected to identify the relevant pharmacokinetic parameters. In addition, this harvested tissue is also assessed for FGFR activation and kinase signaling.

The cardioprotective efficacy of certain FGFs is abrogated in the presence of heparin co-administration. These results identify which FGFs elicit their cardioprotective effect in the presence of heparin, a current standard of care for acute MI. Additive and / or synergistic FGF combinations are also contemplated. A number of FGFs maintain the integrity/function of the myocardium during IR injury, either via direct action on cardiomyocytes and/or indirectly by their angiogenic properties. However, many of the underpinning studies are limited in a clinical scenario s these growth factors were given prior to an ischemic insult. The findings set forth herein (e.g. **Fig. 12A** and **12B****,** **Fig. 13****,** and **Fig. 3B**) suggest that FGF2 and FGF1 are cardioprotective even when treatment occurs immediately upon reperfusion. This timing of FGF therapy mimics the course of treatment in clinical settings of acute MI; since FGFs are heparin-binding proteins; their cardioprotective efficacy in the presence of exogenous heparin for acute MI is unknown. Data set forth herein demonstrates that the cardioprotective action of native FGF2 and FGF1 are abrogated in the presence of heparin co-administration (**Fig. 13** and **Fig. 3B**). Therefore, the present inventors posited that altering the HS-binding region of FGF to affect its binding affinity for heparin/HSPG may change the cardioprotective efficacy of FGF as shown with FGF1^{ΔHBS} **(**Fig. **3B**).

Many proteins, including FGFs, have low stability at physiological temperatures; therefore, their biological application as therapeutics is deemed limited. For example, native FGF1 is remarkably unstable with a half-life for inactivation of only 15 minutes. Data set forth herein supports the conclusion that enhancing FGF thermal stability increases their cardioprotective efficacy. The data in fact demonstrates that enhancing the protein thermal stability of FGF1 improves the efficacy to reduce post-MI infarct size over that of the native form of FGF1 **(**Fig. **18**). Modification to enhance thermal stability of addition FGFs is contemplated to result in additional potent FGF ligands for the treatment of ischemic heart disease.

### Example 9

This example shows that modified FGFs as disclosed herein have enhanced RISK and SAFE signaling even in the presence of heparin. Experiments are undertaken to investigate the role of protein kinases, in particular, those linked with the reperfusion-induced survival kinases (RISK) and survivor activating factor enhancement (SAFE) in FGF-induced cardioprotection.

Data set forth above indicate that ERK1/2 is an important signaling molecule in the cardioprotective effect of FGF1 and FGF 1^{ΔHBS} in a rat model of MI. To determine the molecular mechanism(s) for FGF-mediated cardioprotection, the activity of protein kinases (i.e., RISK and SAFE kinases) that are either involved in FGF signaling or that are implicated in the development of post-conditioning cardioprotection are assessed. The FGFs may induce multiple pathways, some of which may be cardioprotective (PKC-ε, ERK, STAT3, PI3K/Akt). These experiments enable us to decipher the relationship between these FGFs and the protein kinase signaling factors implicated in the genesis and mediation of cardioprotection. Pharmacological manipulations are performed to individually block the protein kinases and measure their changes in activity during reperfusion. The inhibitors are tested with respect to their ability to abrogate protection against myocardial infarction and contractile dysfunction. Therapeutic cardiac signaling of the modified FGF ligands is identified and elucidated.

FGF receptor (FGFR) and protein kinase activation for FGF-induced cardioprotection in absence or presence of heparin is assessed (110 rats). As shown in the above examples, FGFR is activated during native FGF1 or FGF1^{ΔHBS} treatment (**Fig. 6C**). Furthermore, **Fig. 8A** shows that immediately post-FGF1, FGF+heparin, FGF1^{ΔHBS}, or FGF1^{ΔHBS}+heparin treatment, phosphorylation or ERK1/2 was significantly increased compared to saline or heparin only. However, heparin markedly reduced ERK1/2 activation stimulated by FGF1 (**Fig. 8A**). Surprisingly, there was a significantly higher level of phosphorylated ERK1/2 in the FGF1^{ΔHBS}-treated group compared to native FGF1 treatment whether in the absence or presence of heparin (**Fig. 8A**). Neither activation of PKC-α, PKD-δ, nor activation of Akt of the RISK pathway and STAT3 in SAFE signaling were different among the treatment groups or time points (**Figs. 7B-E** and **Figs. 8B-E**). The native and mutant FGFs which elicit a significant cardioprotective effect are assessed for FGFR, ERK, PKC, Akt, and STAT3 activation (i.e. phosphorylation state), well known pathways downstream of FGFR and RISK and SAFE mechanism of cardioprotection, via immunoblotting of whole cell homogenate from left ventricle collected immediately after or 120 minutes post-drug administration as previously described. Phospho-specific and non-phospho-specific antibodies against P13K/Akt, STAT3, MAPKs or PKC isoforms (PKC-α, PKD-δ, PKC-ε) are employed, as activation is indicated as a ratio of phosphorylated protein-total protein. β-actin is used as a loading control. These experiments provide a basis for identifying which kinases to pharmacologically manipulate to identify the primary kinase pathways of FGF-mediated cardioprotection.

### Example 10

This experiment determines the involvement of RISK and SAFE signaling in mutant FGF-induced cardioprotection in the absence or presence of heparin co-administration at reperfusion. (n=166 rats).

Data demonstrates that bisindolylmaleimide (GF109203X), a non-selective PKC inhibitor, abrogates the FGF2-induced cardioprotective effects of reduced myocardial infarct size, suggesting a role for PKC signaling in the cardioprotective effect of FGF2 (**Fig. 17**). Yet, it was unclear (and based on data in **Fig. 8A** and **Fig. 17**) whether each FGF (FGF 1, FGF2, FGF4, FGF5 in native and mutant form) elicits cardioprotection through the same kinase singling. Therefore, to establish the role of specific protein kinases in mutant FGF-induced cardioprotection, the experimental protocol involves treating rats (n = 8 per group) 10 minutes before reperfusion with either vehicle or specific pharmacological agents against PI3K/Akt, JAK/STAT3, ERK1/2 and PKC isoforms (**Table 5**).

**Table 5**

| **Kinase inhibitors and doses to determine RISK and SAFE signaling in FGF-induced cardioprotection** |
|---|
| **PI3K/Akt**: Wortmannin 60 µg/kg i.v.; LY-294002 100 µg/kg i.v. |
| **JAK/STAT3**: AG490 3 mg/kg i.v. |
| **ERK 1/2**: PD-98059 300 µg/kg i.v. ; U0126 200 µg/kg i.v. |
| **PKCα**: Go 6976 300 µg/kg i.v. ; αV fragment 1 mg/kg i.v. |
| **PKCδ**: rottlerin 300 µg/kg i.v. ; δV1.1 1 mg/kg i.v. |
| **PKCε**: εV ₁₋₂ 1 mg/kg i.v. |

Receptors for activated C-kinase (RACK) inhibitors, which are specific peptide fragments to particular PKC isoforms that inhibit the PKC isoforms from translocating and binding to their specific RACK anchoring protein 134, are utilized as a complementary approach to the traditional PKC inhibitors as previously published by our laboratory. Selectivity of these PKC peptide fragments to their specific PKC isoform has been demonstrated in a number of laboratories. The inhibitors to be used against PKC, MAPK, STAT3 or PI3k/Akt are given at the same doses and timing of administration that have been previously been shown to be effective in blocking development of ischemic or pharmacological post-conditioning in the rat (**Table 5**). TAT carrier protein is used as a control for PKC peptide fragments. Kinase inhibition in mutant FGF+heparin studies will also be performed. Although the present investigators and others have published on the kinases involved in FGF-induced cardioprotection, the present study uses native rhFGFs as positive controls. Rats are subjected to 30 minutes ischemia and 2 hours reperfusion and myocardial cell damage and post-ischemic functional recovery is measured after 2 hours reperfusion (**Fig. 2A**). This study elucidated which kinases are involved in FGF-mediated cardioprotection and expect that the modified FGFs will exhibit enhanced protein kinase (both RISK and SAFE) signaling. FGF 1-mediated signals or FGF2 receptor binding are altered, dose-dependently, *in vitro* by heparin. The *in vivo* data demonstrates that heparin does reduce ERK signaling by FGF 1; however, FGF 1^{ΔHBS} enhanced ERK signaling, even in the presence of heparin. Several studies have reported that FGF1 can interact with FGFR and trigger downstream signaling pathways even in the absence of heparan sulfate binding. Moreover, based on the data (FGF1 and ERK1/2 vs. FGF2 and PKC, **Fig. 8A** and **Fig. 17****,** respectively), different FGFs may produce their cardioprotective actions via different protein kinases. Therefore, one or more protein kinases may be involved in one or the other or both endpoints (post-ischemic recovery of function, myocardial infarct size) of cardioprotection. These studies yield important and novel information about FGF-induced cardioprotection and the role of protein kinases in this protective effect.

## Claims

1. One or more Fibroblast growth factors (FGFs) for use in a method of treating or preventing cardiac reperfusion injury in a patient, the method comprising administering the one or more FGFs in conjunction with the onset of cardiac reperfusion therapy;
wherein the one or more FGFs are selected from an FGF engineered to reduce heparin binding affinity, an FGF engineered to increase thermal stability, an FGF engineered to reduce both heparin binding affinity and to increase thermal stability, and combinations thereof, and
wherein the FGF engineered to reduce heparin binding affinity comprises FGF^{ΔHBS}, the FGF engineered to increase thermal stability comprises FGF^{ΔTS}, and the FGF engineered both to reduce heparin binding and to increase thermal stability comprises FGF^{ΔHBS/ΔTS}.

2. The one or more Fibroblast growth factors (FGFs) for use according to claim 1 wherein the patient suffered a myocardial infarction.

3. One or more FGFs for use in a method for the treatment of a patient suffering from or suspected of suffering from a myocardial infarction and about to undergo or undergoing cardiac reperfusion therapy to reduce extent of the myocardial infarction, the method comprising administering the one or more FGFs to the patient in conjunction with the reperfusion therapy;
wherein the one or more FGFs are selected from FGF engineered to reduce heparin binding affinity, FGF engineered to increase thermal stability, FGF engineered both to reduce heparin binding affinity and increase thermal stability, and combinations thereof, and
wherein the FGF engineered to reduce heparin binding affinity comprises FGF^{ΔHBS}, the FGF engineered to increase thermal stability comprises FGF^{ΔTS}, and the FGF engineered both to reduce heparin binding and to increase thermal stability comprises FGF^{ΔHBS/ΔTS}.

4. The one or more FGFs for use according to any one of claims 1 to 3, wherein the method further comprises co-administering Heparin; optionally wherein the Heparin is unfractionated or low molecular weight Heparin.

5. The one or more FGFs for use according to claim 4, wherein "co-administering" comprises administering contemporaneously or in tandem within a circumscribed time frame.

6. The one or more FGFs for use according to claim 5, wherein:
i) "contemporaneously" comprises administering as a single dosage form or as a multiple dosage form;
ii) administering in tandem comprises administering the FGF followed by administering Heparin, or administering Heparin followed by FGF, or any combination thereof; or
iii) administering in tandem comprises administering Heparin, then administering FGF, and, optionally, followed by administering Heparin.

7. The one or more FGFs for use according to any of claims 1-6, wherein:
i) the FGF^{ΔHBS} comprises one or more of FGF 1^{ΔHBS}, FGF2^{ΔHBS}, FGF4^{ΔHBS}, and FGF5^{ΔHBS};
ii) the FGF^{ΔTS} comprises one or more of FGF1^{ΔTS}, FGF2^{ΔTS} , FGF4^{ΔTS}, and FGF5^{ΔTS}; or
iii) the FGF^{ΔHBS/ΔTS} comprises one or more of FGF 1^{ΔHBS/ΔTS}, FGF2^{ΔHBS/ΔTS} FGF4^{ΔHBS/ΔTS}, and FGF5^{ΔHBS/ΔTS}.

8. The one or more FGFs for use according to claim 1 or claim 3:
i) wherein administering "in conjunction with" comprises administering before, at the onset, and subsequent to the onset of reperfusion therapy in the same therapeutic time frame as the reperfusion therapy following an ischemic event in the patient; or
ii) comprising administering the FGF systemically via an enteral or parenteral route.

9. A modified FGF for use in a method of treating a patient who suffered a cardiac ischemic event to restore cardiac function to a pre-ischemic event level of cardiac function, the method comprising administering the modified FGF, and, optionally, heparin,
wherein the administering comprises:
i) administering one or more FGF^{ΔHBS}; or
ii) administering one or more FGF^{ΔHBS} and administering one or more of FGF1, FGF2, FGF1^{ΔHBS} and FGF2^{ΔHBS}

10. The modified FGF for use according to claim 9, wherein:
i) the restored cardiac functioning comprises systolic and/or dystolic function; or
ii) wherein the cardiac ischemic event comprises myocardial infarction.

## Patentansprüche

1. Ein oder mehrere Fibroblastenwachstumsfaktoren (FGFs) zur Verwendung in einem Verfahren zur Behandlung oder
Verhinderung einer kardialen Reperfusionsverletzung bei einem Patienten, das Verfahren umfassend die Verabreichung des einen oder der mehreren FGFs in Verbindung mit dem Beginn der kardialen Reperfusionstherapie;
wobei der eine oder die mehreren FGFs ausgewählt sind aus einem FGF, der konstruiert wurde, um die Heparinbindungsaffinität zu verringern, einem FGF, der konstruiert wurde, um die thermische Stabilität zu erhöhen, einem FGF, der konstruiert wurde, um sowohl die Heparinbindungsaffinität zu verringern als auch die thermische Stabilität zu erhöhen, und Kombinationen davon, und
wobei der FGF, der zur Verringerung der Heparinbindungsaffinität entwickelt wurde, FGF^{ΔHBS} umfasst, der FGF, der zur Erhöhung der thermischen Stabilität entwickelt wurde, FGF^{ΔTS} umfasst, und der FGF, der sowohl zur Verringerung der Heparinbindung als auch zur Erhöhung der thermischen Stabilität entwickelt wurde, FGF^{ΔHBS/ΔTS} umfasst.

2. Ein oder mehrere Fibroblastenwachstumsfaktoren (FGFs) zur Verwendung nach Anspruch 1, wobei der Patient einen Myokardinfarkt erlitten hat.

3. Ein oder mehrere FGFs zur Verwendung in einem Verfahren zur Behandlung eines Patienten, der an einem Myokardinfarkt leidet oder bei dem der Verdacht besteht, dass er an einem Myokardinfarkt leidet, und der sich einer kardialen Reperfusionstherapie unterziehen soll oder unterzieht, um das Ausmaß des Myokardinfarkts zu verringern, wobei das Verfahren die Verabreichung des einen oder der mehreren FGFs an den Patienten in Verbindung mit der Reperfusionstherapie umfasst;
wobei der eine oder die mehreren FGFs ausgewählt sind aus FGFs, die konstruiert wurden, um die Heparinbindungsaffinität zu verringern, FGFs, die konstruiert wurden, um die thermische Stabilität zu erhöhen, FGFs, die konstruiert wurden, um sowohl die Heparinbindungsaffinität zu verringern als auch die thermische Stabilität zu erhöhen, und Kombinationen davon, und
wobei der FGF, der zur Verringerung der Heparinbindungsaffinität entwickelt wurde, FGF^{ΔHBS} umfasst, der FGF, der zur Erhöhung der thermischen Stabilität entwickelt wurde, FGF^{ΔTS} umfasst,
und der FGF, der konstruiert wurde, um sowohl die Heparinbindung zu verringern als auch die thermische Stabilität zu erhöhen, FGF^{ΔHBS/ΔTS} umfasst.

4. Ein oder mehrere FGFs zur Verwendung nach einem der Ansprüche 1 bis 3, wobei das Verfahren ferner die gleichzeitige Verabreichung von Heparin umfasst; wobei es sich bei dem Heparin optional um unfraktioniertes oder niedermolekulares Heparin handelt.

5. Ein oder mehrere FGFs zur Verwendung nach Anspruch 4, wobei "gemeinsame Verabreichung" die gleichzeitige oder gemeinsame Verabreichung innerhalb eines umschriebenen Zeitrahmens umfasst.

6. Ein oder mehrere FGFs zur Verwendung nach Anspruch 5, wobei:
i) "gleichzeitig" umfasst die Verabreichung als Einzeldarreichungsform oder als Mehrfachdarreichungsform;
ii) gemeinsame Verabreichung umfasst die Verabreichung von FGF, gefolgt von der Verabreichung von Heparin, oder die Verabreichung von Heparin, gefolgt von FGF, oder eine beliebige Kombination davon; oder
iii) gemeinsame Verabreichung umfasst die Verabreichung von Heparin, dann die Verabreichung von FGF und optional die anschließende Verabreichung von Heparin.

7. Ein oder mehrere FGFs zur Verwendung nach einem der Ansprüche 1 bis 6, wobei:
i) der FGF^{ΔHBS} eines oder mehrere von FGF1^{ΔHBS}, FGF2^{ΔHBS} , FGF4^{ΔHBS} und FGF5^{ΔHBS} umfasst;
ii) der FGF^{ΔTS} eines oder mehrere von FGF1^{ΔTS}, FGF2^{ΔTS}, FGF4^{ΔTS} und FGF5^{ΔTS} umfasst; oder
iii) der FGF^{ΔHBS/ΔTS} eines oder mehrere von FGF1^{ΔHBS/ΔTS}, FGF2^{ΔHBS/ΔTS} FGF4^{ΔHBS/ΔTS},und FGF5^{ΔHBS/ΔTS} umfasst.

8. Ein oder mehrere FGFs zur Verwendung nach Anspruch 1 oder 3:
i) wobei die Verabreichung "in Verbindung mit" die Verabreichung vor, zu Beginn und nach dem Beginn der Reperfusionstherapie im gleichen therapeutischen Zeitrahmen wie die Reperfusionstherapie nach einem ischämischen Ereignis in dem Patienten umfasst; oder
ii) umfassend die systemische Verabreichung des FGF über einen enteralen oder parenteralen Weg.

9. Modifizierter FGF zur Verwendung in einem Verfahren zur Behandlung eines Patienten, der ein ischämisches Herzereignis erlitten hat, um die Herzfunktion auf eine Stufe der Herzfunktion vor dem ischämischen Ereignis wiederherzustellen, wobei das Verfahren die Verabreichung des modifizierten FGF und optional Heparin umfasst,
wobei die Verabreichung Folgendes umfasst:
i) Verabreichen eines oder mehrerer FGF^{ΔHBS}; oder
ii) Verabreichen eines oder mehrerer FGF^{ΔHBS} und Verabreichen eines oder mehrerer von FGF1, FGF2, FGF1^{ΔHBS} und FGF2^{ΔHBS}.

10. Modifizierter FGF zur Verwendung nach Anspruch 9, wobei:
i) die wiederhergestellte Herzfunktion eine systolische und/oder dystolische Funktion umfasst;; oder
ii) wobei das ischämische Herzereignis einen Myokardinfarkt umfasst.

## Revendications

1. Facteur ou facteurs de croissance des fibroblastes (FGF) à utiliser dans un procédé de traitement ou
de prévention de lésion de reperfusion cardiaque chez un patient, le procédé comprenant l'administration du ou des FGF en conjonction avec le début de la thérapie de reperfusion cardiaque ;
dans lesquels le ou les FGF sont choisis parmi un FGF conçu pour réduire l'affinité de liaison à l'héparine, un FGF conçu pour augmenter la stabilité thermique, un FGF conçu à la fois pour réduire l'affinité de liaison à l'héparine et pour augmenter la stabilité thermique, et leurs combinaisons, et
dans lesquels le FGF conçu pour réduire l'affinité de liaison à l'héparine comprend le FGF^{ΔHBS}, LE FGF conçu pour augmenter la stabilité thermique comprend le FGF^{ΔTS}, et le FGF conçu à la fois pour réduire la liaison à l'héparine et pour augmenter la stabilité thermique comprend le FGF^{ΔHBS/ΔTS}.

2. Facteur ou facteurs de croissance des fibroblastes (FGF) à utiliser selon la revendication 1, dans lesquels le patient a souffert d'un infarctus du myocarde.

3. Facteur ou facteurs de croissance des fibroblastes à utiliser dans un procédé de traitement d'un patient souffrant ou suspecté de souffrir d'un infarctus du myocarde et sur le point de subir ou subissant une thérapie de reperfusion cardiaque pour réduire l'étendue de l'infarctus du myocarde, le procédé comprenant l'administration du ou des FGF au patient en conjonction avec la thérapie de reperfusion ;
dans lequel le ou les FGF sont choisis parmi le FGF conçu pour réduire l'affinité de liaison à l'héparine, le FGF conçu pour augmenter la stabilité thermique, le FGF conçu à la fois pour réduire l'affinité de liaison à l'héparine et pour augmenter la stabilité thermique, et leurs combinaisons, et
dans lesquels le FGF conçu pour réduire l'affinité de liaison à l'héparine comprend le FGF^{ΔHBS}, LE FGF conçu pour augmenter la stabilité thermique comprend le FGF^{ΔTS},
et le FGF conçu à la fois pour réduire la liaison à l'héparine et pour augmenter la stabilité thermique comprend le FGF^{ΔHBS/ΔTS}.

4. Facteur ou facteurs de croissance des fibroblastes à utiliser selon l'une quelconque des revendications 1 à 3, dans lesquels le procédé comprend en outre la co-administration de l'héparine ; éventuellement dans lesquels l'héparine est une héparine non fractionnée ou de faible poids moléculaire.

5. Facteur ou facteurs de croissance des fibroblastes à utiliser selon la revendication 4, dans lesquels la « co-administration » comprend l'administration simultanée ou en tandem dans un laps de temps circonscrit.

6. Facteur ou facteurs de croissance des fibroblastes à utiliser selon la revendication 5, dans lesquels :
i) « simultanément » comprend l'administration sous une forme de dosage unique ou sous une forme de dosage multiple ;
ii) l'administration en tandem comprend l'administration du FGF suivie de l'administration de l'héparine, ou l'administration de l'héparine suivie du FGF, ou leur combinaison quelconque ; ou
iii) l'administration en tandem comprend l'administration de l'héparine, puis l'administration du FGF et, éventuellement, suivie de l'administration de l'héparine.

7. Facteur ou facteurs de croissance des fibroblastes à utiliser selon l'une quelconque des revendications 1 à 6, dans lesquels :
i) le FGF^{ΔHBS} comprend un ou plusieurs parmi FGF1^{ΔHBS}, FGF2^{ΔHBS}. FGF4^{ΔHBS} et FGF5^{ΔHBS} ;
ii) le FGF^{ΔTS} comprend un ou plusieurs parmi FGF1^{ΔTS}, FGF2^{ΔTS}, FGF4^{ΔTS} et FGF5^{ΔTS} ; ou
iii) le FGF^{ΔHBS/ΔTS} comprend un ou plusieurs parmi FGF1^{ΔHBS/ΔTS}, FGF2^{ΔHBS/ΔTS}, FGF4^{ΔHBS/ΔTS} et FGF5^{ΔHBS/ΔTS}.

8. Facteur ou facteurs de croissance des fibroblastes à utiliser selon la revendication 1 ou la revendication 3 :
i) dans lesquels l'administration « en conjonction avec » comprend l'administration avant, au début et après le début de la thérapie de reperfusion dans le même laps de temps thérapeutique que la thérapie de reperfusion à la suite d'un événement ischémique chez le patient ; ou
ii) comprenant l'administration du FGF par voie systémique par l'intermédiaire d'une voie entérale ou parentérale.

9. FGF modifié à utiliser dans un procédé de traitement d'un patient ayant subi un événement d'ischémie cardiaque pour restaurer la fonction cardiaque à un niveau antérieur à l'événement d'ischémie, le procédé comprenant l'administration du FGF modifié et, éventuellement, de l'héparine,
dans lequel l'administration comprend :
i) l'administration d'un ou plusieurs FGF^{ΔHBS} ; ou
ii) l'administration d'un ou plusieurs FGF^{ΔHBS} et l'administration d'un ou plusieurs parmi FGF1, FGF2, FGF1^{ΔHBS} et FGF2^{ΔHBS}.

10. FGF modifié à utiliser selon la revendication 9, dans lequel :
i) la fonction cardiaque restaurée comprend la fonction systolique et/ou dystolique ; ou
ii) dans lequel l'événement ischémique cardiaque comprend l'infarctus du myocarde.
